# EUROPEAN PATENT SPECIFICATION

(11) **EP 2 320 953 B1**
(45) Date of publication and mention of the grant of the patent: **15.07.2015**
(21) Application number: 09805654.2
(22) Date of filing: 10.08.2009
(51) Int. Cl.: C12N 15/87, C12N 15/88, A61K 48/00

(54) **LONG-ACTING DNA DENDRIMERS AND METHODS THEREOF**
DNA-DENDRIMERE MIT LANGZEITWIRKUNG UND VERFAHREN DAFÜR
DENDRIMÈRES D'ADN À LONGUE DURÉE D'ACTION ET MÉTHODES AFFÉRENTES

(30) Priority: 08.08.2008 US 188318 P
(43) Date of publication of application: 18.05.2011
(60) Divisional application: 13178199.9
(73) Proprietor: Genisphere, LLC, Hatfield, PA 19440 (US)
(72) Inventor: KADUSHIN, James, M., Gilbertsville,PA 19525 (US); GETTS, Robert, C., Collegeville,PA 19426 (US)
(74) Representative: Herzog, Fiesser & Partner Patentanwälte PartG mbB
(86) International application number: PCT/US2009/053264
(87) International publication number: WO 2010/017544

(56) References cited:
- WO-A1-2008/147526
- Kadushin J.M. & Getts L.A.: "Enhancement of sensitivity in Luminex protein detection assays via dendrimer dependent signal amplification", Luminex Planet xMAP conference, April 25-27, 2005 (Austin TX). , 2005, XP002617108, Retrieved from the Internet: URL:http://www.genisphere.com/pdf/Genisphe re_Luminex_Planet_xMAP_042505.pdf [retrieved on 2011-01-17]
- Genisphere: "Starfish (TM) fluorescent in situ detection kit", , 2000, XP002617103, Retrieved from the Internet: URL:www.genisphere.com [retrieved on 2011-01-17]
- PATIL M.L. ET AL.: "Surface-modified and internally cationic polyamidoamine dendrimers for efficient siRNA delivery", BIOCONJUGATE CHEM., vol. 19, 25 June 2008 (2008-06-25), pages 1396-1403, XP002617104,
- Genisphere: An introduction to 3DNA technology , 2005, XP002617105, Retrieved from the Internet: URL:www.genisphere.com [retrieved on 2011-01-17]
- Dirk Haussecker: "Is dendrimer-siRNA delivery reaching critical mass?", Blog , 30 September 2009 (2009-09-30), XP002617106, Retrieved from the Internet: URL:http://rnaitherapeutics.blogspot.com/2 009/09/is-dendrimer-sirna-delivery-reachin g.html [retrieved on 2010-01-17]

### SEQUENCE LISTING

<110> Genisphere
<120> LONG-ACTING DNA DENDRIMERS AND METHODS THEREOF
<130> P-73374-PC
<150> US 61/188,318
   <151> 2008-08-08
<160> 19
<170> PatentIn version 3.5
<210> 1
   <211> 19
   <212> RNA
   <213> Artificial Sequence
<220>
   <223> SSB SiRNA, RNA antisense strand
<400> 1
   uuaaagucug uugucagcc 19
<210> 2
   <211> 19
   <212> RNA
   <213> Artificial Sequence
<220>
   <223> SSB SiRNA, RNA sense strand
<400> 2
   ggcugacaac agacuuaaa 19
<210> 3
   <211> 19
   <212> RNA
   <213> Artificial Sequence
<220>
   <223> Negative Control siRNA, RNA antisense strand
<400> 3
   acgugacacg uucggagaa 19
<210> 4
   <211> 19
   <212> RNA
   <213> Artificial Sequence
<220>
   <223> Negative Control siRNA, RNA sense strand
<400> 4
   uucuccgaac gugucacgu 19
<210> 5
   <211> 18
   <212> DNA
   <213> Artificial Sequence
<220>
   <223> SSB with 16 base DNA linker sequence, DNA sense strand
<400> 5
   ttccgttgac atctcgta 18
<210> 6
   <211> 23
   <212> DNA
   <213> Artificial Sequence
<220>
   <223> SSB with 21 base DNA linker sequence, DNA sense strand
<400> 6
   ttccgttgac atctcgtaga ttt 23
<210> 7
   <211> 28
   <212> RNA
   <213> Artificial Sequence
<220>
   <223> SSB with 26 base DNA linker sequence, DNA sense strand
<400> 7
   ttccgttgac atctcgtaga tttgaatt 28
<210> 8
   <211> 31
   <212> DNA
   <213> Artificial Sequence
<220>
   <223> Cap03 abiotic sequence for structural "capture sequences" attached to DNA dendrimer
<400> 8
   tccaccttag agtacaaacg gaacacgaga a 31
<210> 9
   <211> 31
   <212> DNA
   <213> Artificial Sequence
<220>
   <223> Cap03 abiotic anti-sense sequence attached to DNA dendrimer
<400> 9
   ttctcgtgtt ccgtttgtac tctaaggtgg a 31
<210> 10
   <211> 21
   <212> DNA
   <213> Artificial Sequence
<220>
   <223> siRNAs Antisense strand without dendrimer attachment sequence
<400> 10
   uuaaagucug uugucagccg g 21
<210> 11
   <211> 21
   <212> DNA
   <213> Artificial Sequence
<220>
   <223> siRNAs sense strand without dendrimer attachment sequence
<400> 11 21
   ggcugacaac agacuuuaat t 21
<210> 12
   <211> 21
   <212> DNA
   <213> Artificial Sequence
<220>
   <223> Negative Control antisense SiRNA
<400> 12
   acgugacacg uucggagaat t 21
<210> 13
   <211> 21
   <212> DNA
   <213> Artificial Sequence
<220>
   <223> Negative Control sense strand SiRNA
<400> 13
   uucuccgaac gugucacgut t 21
<210> 14
   <211> 47
   <212> DNA
   <213> Artificial Sequence
<220>
   <223> Negative Control (no mRNA target) with 26 base linker sense strand sequence
<400> 14
   uucuccgaac gugucacgut tccgttgaca tctcgtagat ttgaatt 47
<210> 15
   <211> 68
   <212> DNA
   <213> Artificial Sequence
<220>
   <223> SSB with 26 base DNA linker sense strand sequence
<400> 15
   ggcugacaac agacuuuaad tdtdcdcdgd tdtdgdadcd adtdcdtdcd gdtdadgdad 60
   tttgaatt 68
<210> 16
   <211> 37
   <212> DNA
   <213> Artificial sequence
<220>
   <223> Condition #2: SSB with 16 base DNA linker sense strand sequence
<400> 16
   ggcugacaac agacuuuaat tccgttgaca tctcgta 37
<210> 17
   <211> 21
   <212> DNA
   <213> Artificial Sequence
<220>
   <223> Negative Control (no mRNA target) with 16 base linker anti-sense strand sequence
<400> 17
   acgugacacg uucggagaat t 21
<210> 18
   <211> 37
   <212> DNA
   <213> Artificial Sequence
<220>
   <223> Negative Control (no mRNA target) with 16 base linker sense strand sequence
<400> 18
   uucuccgaac gugucacgut tccgttgaca tctcgta 37
<210> 19
   <211> 42
   <212> DNA
   <213> Artificial Sequence
<220>
   <223> Condition #3: SSB with 21 base DNA linker sense strand sequence
<400> 19
   uucuccgaac gugucacgut tccgttgaca tctcgtagat tt 42

## Description

### FIELD OF INVENTION

A composition including a DNA dendrimer and a siRNA molecule attached thereto and methods of preparing, protecting and delivering the same are provided.

### BACKGROUND OF THE INVENTION

Dendritic molecules are repeatedly branched species that are characterized by structural perfection. This is based on the evaluation of both symmetry and polydispersity. The field of dendritic molecules can roughly be divided into low-molecular weight and high-molecular weight species. The first category includes dendrimers and dendrons, and the second includes dendronised polymers, hyperbranched polymers and brush-polymers.

The first dendrimers were synthesized divergently and in 1990 a convergent synthesis was introduced. Dendrimers then experienced an explosion of scientific interest because of their unique molecular architecture.

DNA dendrimers are complex, highly branched molecules built from interconnected natural or synthetic DNA subunits. A DNA dendrimer is constructed from partially double stranded DNA monomers, each of which is made from two single stranded DNA molecules that share a region of sequence complementarity located in the central portion of each strand. Monomers are combined during the manufacturing process to prepare DNA dendrimers of different sizes and shapes. In order to prevent DNA dendrimers from falling apart over time, chemical "spot welds" are added to the growing assembly during the process using UV light via the intercalation and activation of psoralen cross-linkers.

Multi-molecular scaffold devices, including DNA dendrimers, may be useful as cellular transfection, imaging, and drug delivery agents. Specifically, DNA dendrimers are bound with targeting devices (e.g. an antibody specific for a cell surface feature capable of eliciting an cellular endocytotic internalization event) and can bind to surface features on cells targeted to receive the delivery of a cargo (e.g. a drug). Cargos may be passively associated with the targeted DNA dendrimer and enter the cell simply by spatial association with the dendrimer, or cargos may be directly bound to the dendrimer via a number of attachment strategies.

The typical response elicited by siRNA molecules is referred to as mRNA knockdown or reduction of steady-state mRNA levels, with the sequence of the siRNA molecule determining which gene or genes are to be targeted for knockdown.

### SUMMARY OF THE INVENTION

In one embodiment of the invention, the present invention provides a composition comprising a DNA dendrimer attached to a siRNA molecule.

In another embodiment of the invention, the present invention provides a method of preparing a composition comprising a DNA dendrimer and a siRNA molecule, as characterized in the claims comprising the step of attaching a siRNA to a DNA dendrimer (a) via a disulfide bridging bond; (b) via the use of NHS ester dependent condensation reaction; (c) via the use of heterobifunctional cross linking reaction; (d) via direct or indirect hybridization of the siRNA to DNA dendrimer sequence; or (e) via the use of polycationic compounds to bridge siRNA molecule to the DNA dendrimer via charge-charge interactions.

The present disclosure also provides a method of protecting a siRNA molecule against degradation, comprising the step of attaching (a) a siRNA molecule and (b) a protein, digoxigenin, cholesterol, a primary amine, a hydrocarbon spacer of length of 3 to 120 carbons, a PEG molecule, biotin, a biotin derivative, fluorescein or a fluorescein derivative, or any combination thereof, to a DNA dendrimer, thereby protecting a siRNA molecule against degradation.

The present disclosure also provides a method of protecting a DNA dendrimer against degradation in a bodily fluid, comprising the step of attaching to the DNA dendrimer a protein, digoxigenin, cholesterol, a primary amine, a hydrocarbon spacer of length of 3 to 120 carbons, a molecule containing a PEG moiety, biotin or a biotin derivative, fluorescein or a fluorescein derivative, or any combination thereof, thereby protecting a DNA dendrimer against nuclease degradation in a bodily fluid.

The present disclosure also provides a method of delivering a DNA dendrimer into a bodily fluid, comprising the step of attaching to the DNA dendrimer a protein, a molecule containing a PEG moiety, biotin or a biotin derivative, fluorescein or a fluorescein derivative, or any combination, thereby delivering a DNA dendrimer into a bodily fluid.

### BRIEF DESCRIPTION OF THE DRAWINGS

FIG. 1 is an illustration of the DNA strands and monomers used for preparation of dendrimer components.
FIG. 2 is an illustration of the DNA dendrimer assembly steps, showing the sequential growth of the DNA dendrimers (as layers) as various monomers are added.

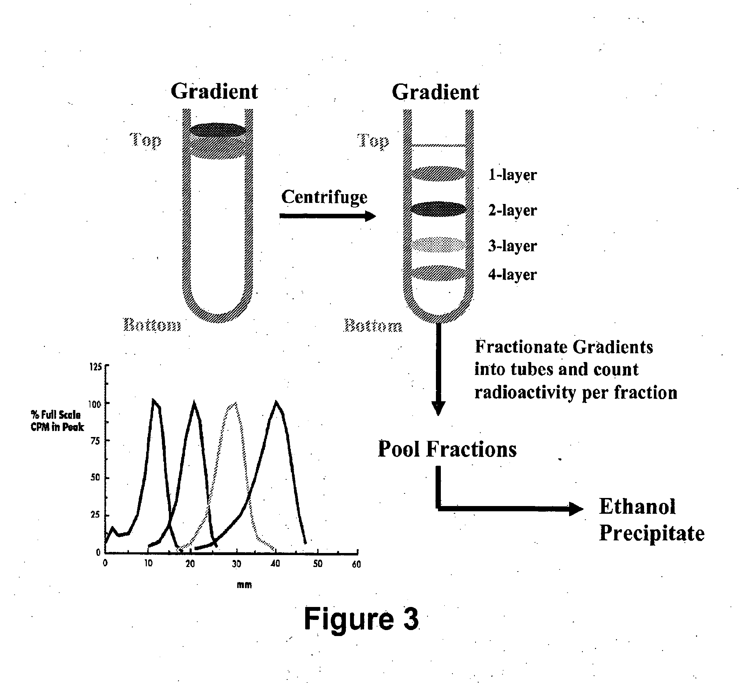

FIG. 3 is an illustration of DNA dendrimers' purification steps.

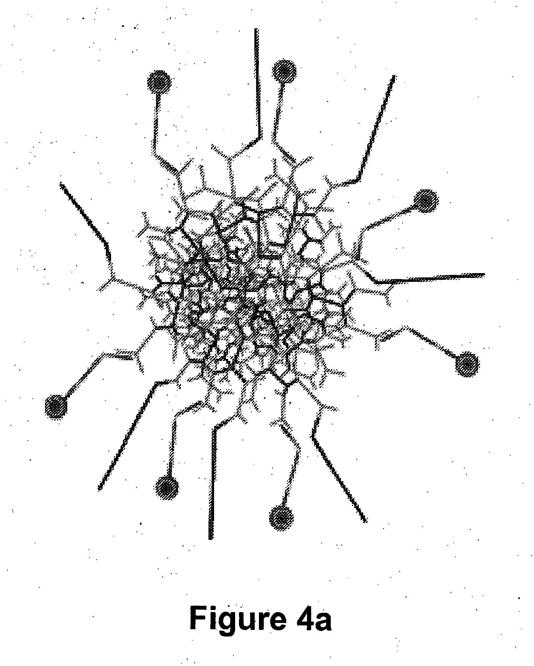

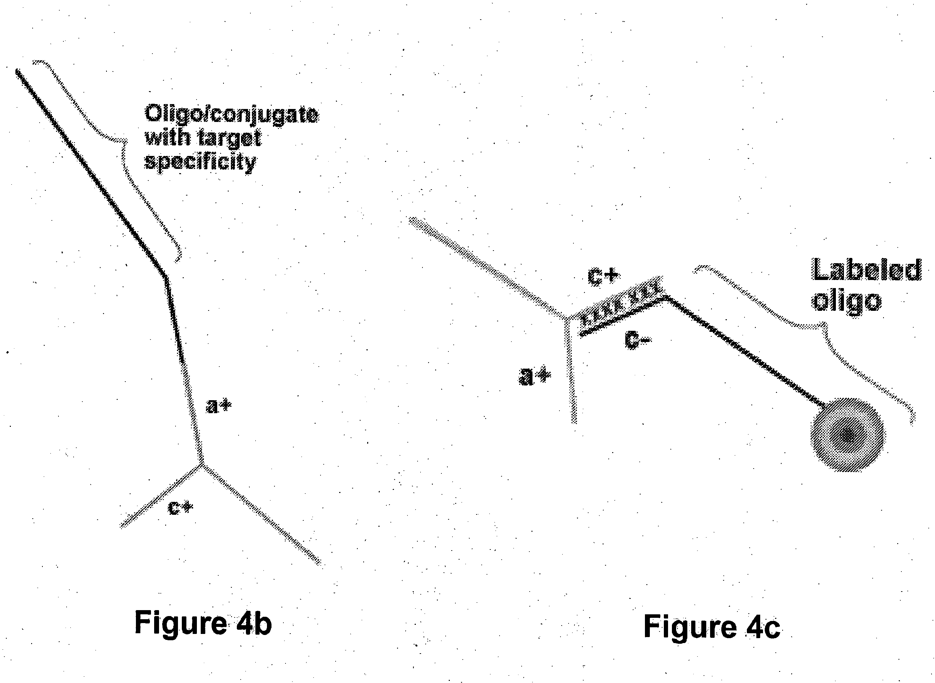

FIG. 4 is an illustration of DNA dendrimers comprising attachments of specific oligo and signal molecules (4a); enlarged image of the target specificity bound to the dendrimer via oligo ligation (4b); and enlarged image of the covalent attachment of a labeled oligo to the arm of the dendrimer (4c).
FIG. 5 is a micrograph of a gel showing the degradation of: the non-modified four layer DNA dendrimer, 0-120min (5a); and the modified four layer DNA dendrimer 0-120min (resistant)(5b).
FIG. 6 is a micrograph of a gel showing the degradation of: the non-modified four layer DNA dendrimer 0-960min (6a); and the modified four layer DNA dendrimer 0-960 minutes (resistant) (6b).

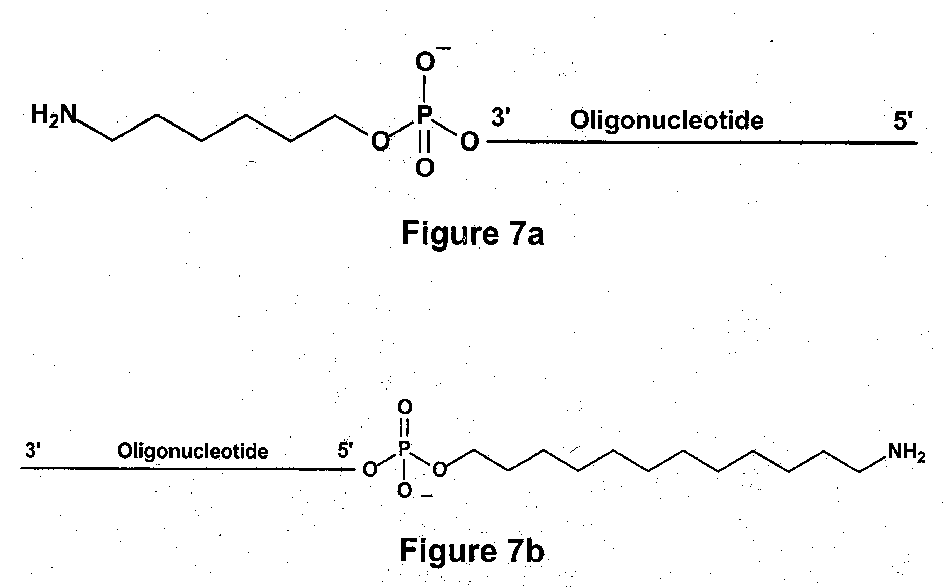

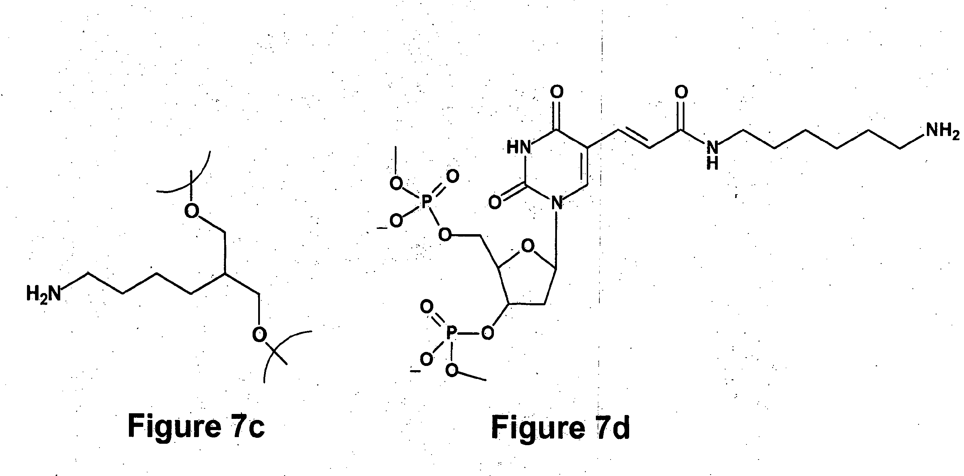

FIG. 7. Chemical structures of: 6 carbon amino linker amidite structure (for DNA oligo synthesis) (7A); 12 carbon amino linker amidite structure (for DNA oligo synthesis) (7B); 7 carbon internal amino linker amidite structure (for DNA oligo synthesis) (7C); dT internal amino linker amidite structure (for DNA oligo synthesis) (7D).
FIG. 8. Chemical structures of NHS esters; Cy™3 NHS ester (8a) Excitation max = 548 nm, Emission max = 562 nm; Cy5 NHS ester (8b) Excitation max = 646 nm, Emission max = 664 nm.
FIG 9. Chemical structure of Oyster 550-D (9a); and Chemical structure of Oyster 650-D(9b).

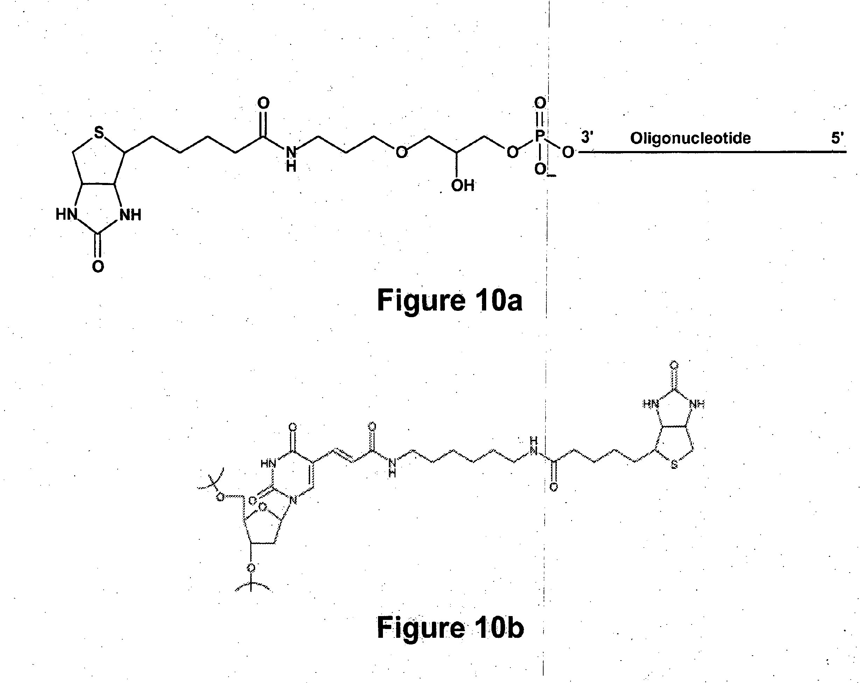

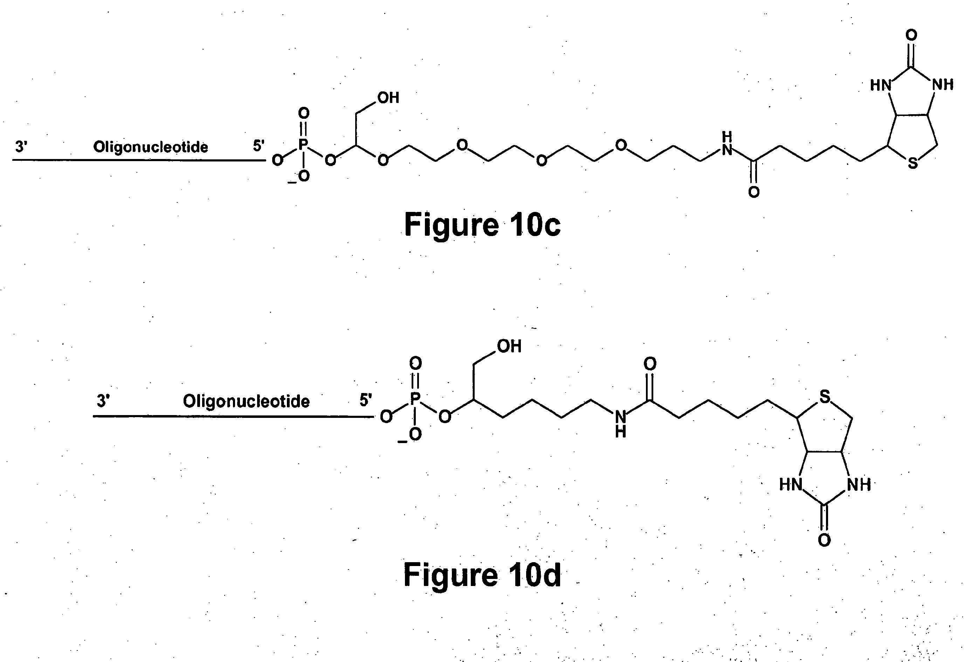

FIG. 10. Chemical structures of: Biotin-BB-CPG amidite structure (for DNA oligo synthesis) (10a); Structure of biotin-dT amidite (for DNA oligo synthesis) (10b); Structure of biotin-TEG amidite (for DNA oligo synthesis) C9O4 spacer (10c); Structure of biotin-TEG amidite (for DNA oligo synthesis) C4 spacer (10d).

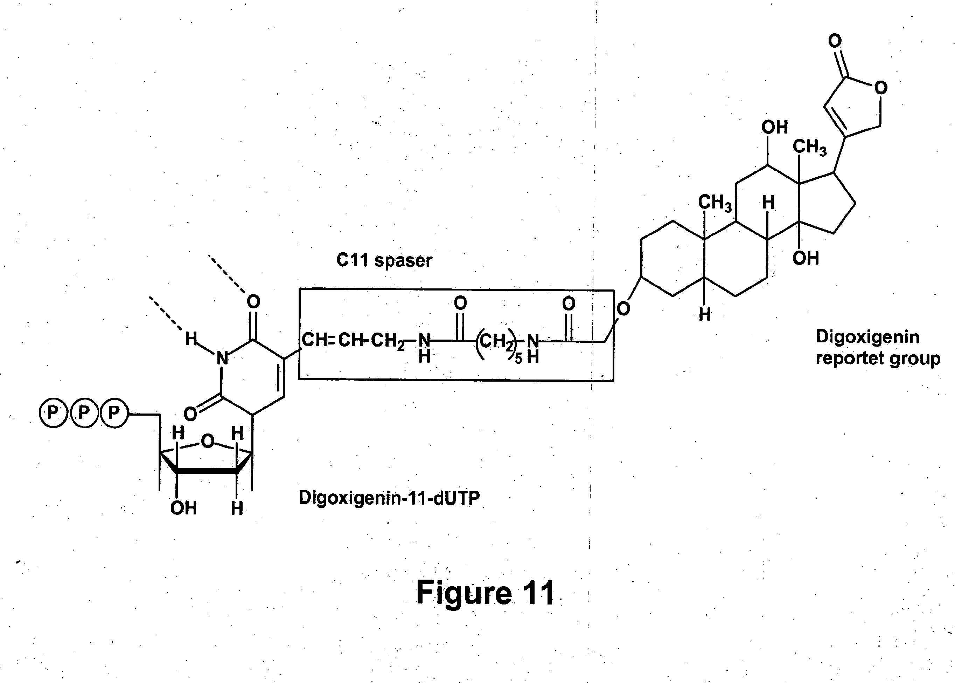

FIG 11. Chemical structures of digoxigenin 11-UTP and the reporter.

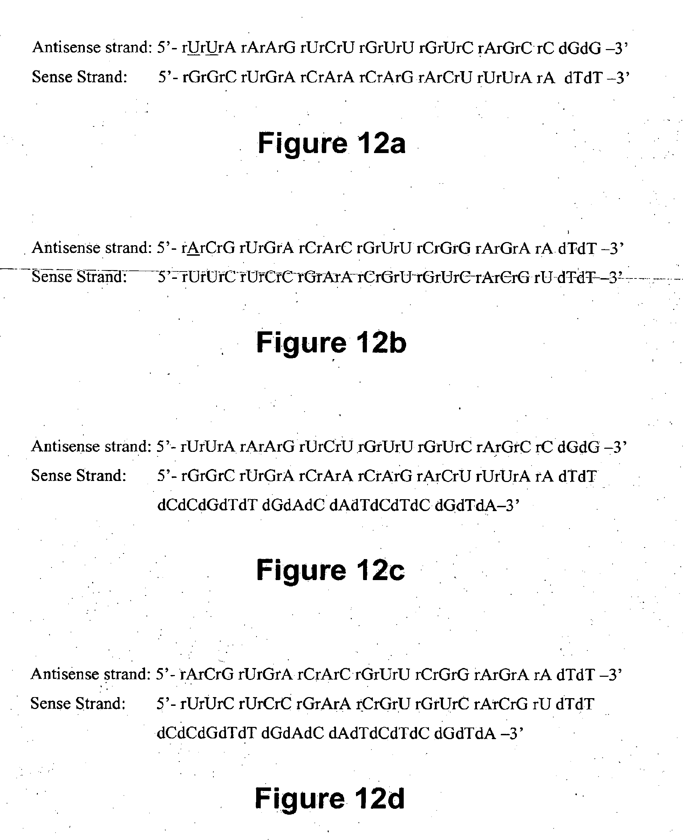

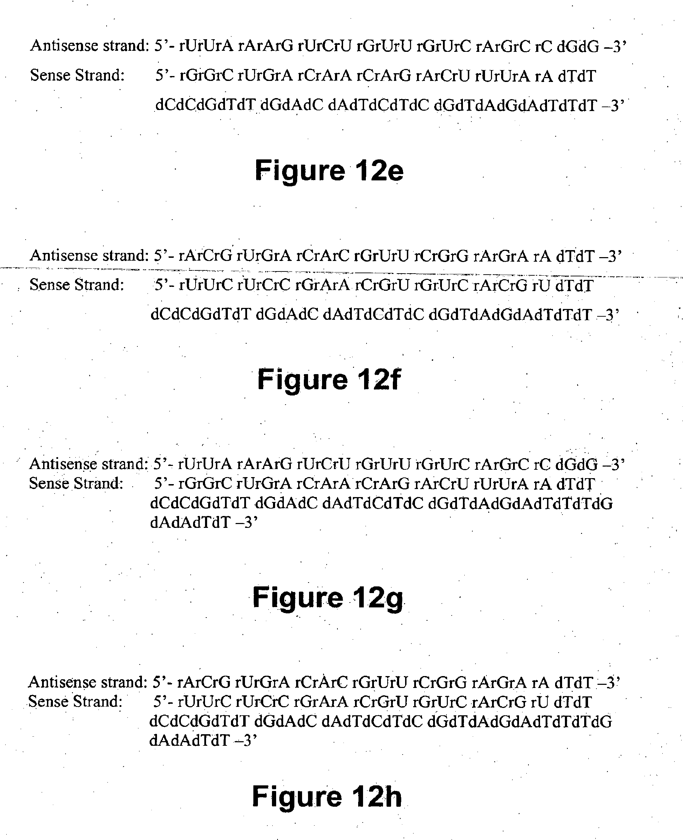

FIG 12. The siRNA RNA-DNA hybrid constructs utilized with the DNA dendrimers: the sequences of the antisense and sense strands of the SSB siRNAs (12a) and the negative control siRNA (12b); the sequences of the antisense and sense strands of the SSB with 16 base DNA linker sequence (12c) and the negative control (no mRNA target) with 16 base linker sequence (12d); the sequences of the antisense and sense strands of the SSB with 21 base DNA linker sequence (12e) and the negative control (no mRNA target) with 21 base linker sequence (12f); and the sequences of the antisense and sense strands of the SSB with 26 base DNA linker sequence (12g) and the negative control (no mRNA target) with 26 base linker sequence (12h).

### DETAILED DESCRIPTION OF THE INVENTION

In one embodiment of the invention, the present invention provides a composition comprising a DNA dendrimer attached to a siRNA molecule. In another embodiment of the invention, the composition further comprises a protein, a fluorescein or a fluorescein derivative such as but not limited to FITC, a PEG molecule, biotin, a biotin derivative, or any combination thereof further attached to the DNA dendrimer. Surprisingly, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, a FITC, a PEG molecule, biotin, a biotin derivative, or any combination thereof to a DNA dendrimer protected the DNA dendrimer from body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof to a DNA dendrimer protected the DNA dendrimer and the siRNA molecule attached thereto from body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein or fluorescein derivative, or any combination thereof to a DNA dendrimer, protected the DNA dendrimer and the siRNA molecule attached thereto body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein or fluorescein derivative, or any combination thereof to a DNA dendrimer protected the DNA dendrimer and the siRNA molecule attached thereto from nuclease dependent degradation.

The nuclease may be a protein DNase. The nuclease may be an exogenous DNase. the nuclease may be any different protein DNases known to one of skill in the art. Serum degradation may be serum nuclease degradation. Surprisingly, it was found that attaching a protein, a FITC, a PEG molecule, biotin, a biotin derivative, fluorescein or a fluorescein derivative, or any combination thereof to a DNA dendrimer stabilizes the DNA dendrimer and the siRNA molecule attached thereto against serum degradation.

The composition may comprise a protective group, which protects the composition against degradation. The composition may comprise a protective group, which protects the composition against degradation in body fluids such as serum, blood plasma, etc. The composition may comprise a protective group, which protects the composition against nuclease dependent degradation. The protective group may be a compound or a molecule, which stabilizes the DNA dendrimer. The protective group is a compound or a molecule which protects the DNA dendrimer against degradation. The protective group is a compound or a molecule which protects the DNA dendrimer against degradation in a body fluid. The protective group is a compound or a molecule which protects the DNA dendrimer against degradation in serum.

The stabilized composition as described herein may be stable in serum, in a composition comprising serum, in blood, or any other body fluid for at least 0.3 hours, at least 0.5 hours, at least 0.7 hours, at least 1 hour, at least 1.5 hours, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, least 14 hours, at least 15 hours, at least 16 hours, at least 20 hours, at least 24 hours, at least 30 hours, at least 36 hours, at least 48 hours, at least 60 hours, at least 72 hours.

The stabilized composition as described herein may be stable in serum, in a composition comprising serum, in blood, or any other body fluid for 1-24 hours, for 1-20 hours, for 5-15 hours, for 10-16 hours.

In another embodiment of the invention, the invention further provides a composition comprising an antibody or a fragment thereof and a siRNA molecule attached to a DNA dendrimer. The antibody may be a conjugated antibody, a monoclonal antibody, a polyclonal antibody, a single-chain Fv fragment (SCFV) antibody, or any antibody or a conjugated antibody known to one of skill in the art.

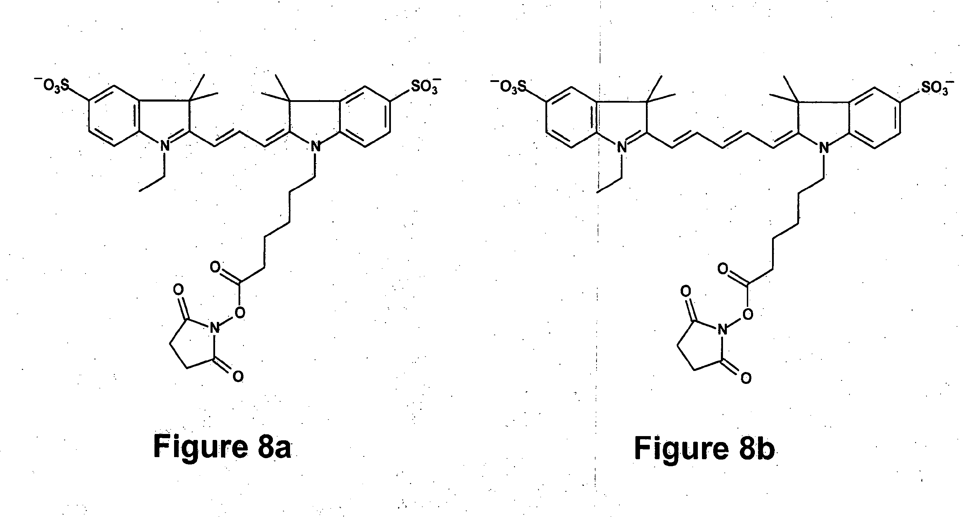

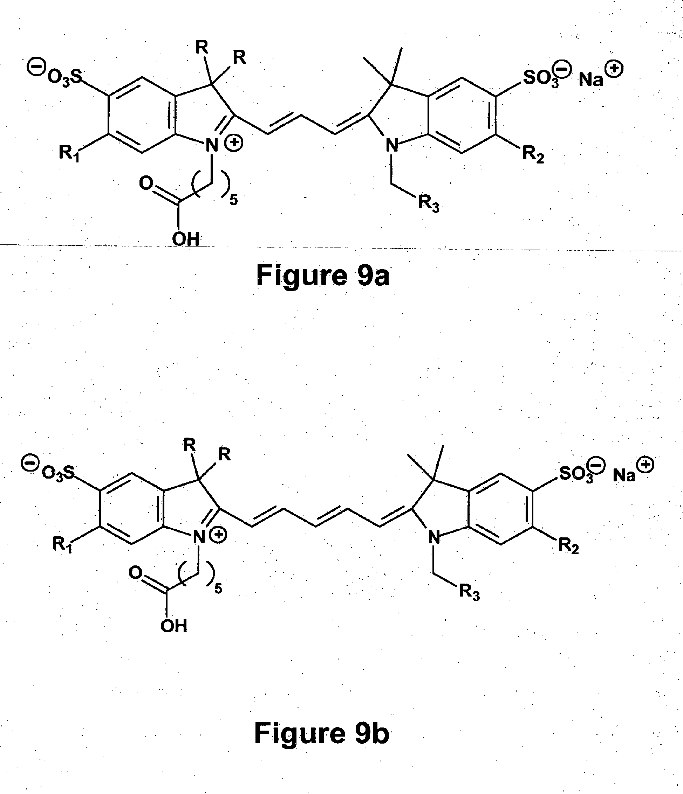

The disclosure further provides a composition comprising a dye molecule and a siRNA molecule attached to a DNA dendrimer. The disclosure further provides a composition comprising a dye molecule comprising a detectable label attached to a linker or spacer (figure 8 and figure 9) and a siRNA molecule attached to a DNA dendrimer. The disclosure further provides a composition comprising a fluorophore and a siRNA molecule attached to a DNA dendrimer. In an embodiment of the invention, the invention provides a composition comprising fluorescein and a siRNA molecule attached to a DNA dendrimer. In another embodiment of the invention, the invention further provides a composition comprising fluorescein isothiocyanate (FITC) and a siRNA molecule attached to a DNA dendrimer. In another embodiment of the invention, FITC is referred as a fluorescein, derivative.

The disclosure further provides a composition comprising a molecule comprising an ureido (tetrahydroimidizalone) ring and a siRNA molecule attached to a DNA dendrimer. The disclosure further provides a composition comprising a molecule comprising a tetrahydrothiophene ring and a siRNA molecule attached to a DNA dendrimer. The disclosure further provides a composition comprising a molecule comprising valeric acid substituent and a siRNA molecule attached to a DNA dendrimer. The disclosure further provides a composition comprising a molecule serving as a cofactor in the metabolism of fatty acids and a siRNA molecule attached to a DNA dendrimer. The disclosure further provides a composition comprising a molecule serving as a cofactor in the metabolism of leucine and a siRNA molecule attached to a DNA dendrimer. In an embodiment of the invention, the invention provides a composition comprising biotin and a siRNA molecule attached to a DNA dendrimer.

The term "biotin" includes known biotin derivatives. A biotin derivative binds strepavidin. In another embodiment of the invention, the term "fluorescein" includes known fluorescein derivatives.

The disclosure further provides a composition as described herein, further comprising serum. The disclosure further provides a composition as described herein, further comprising blood. The disclosure further provides a composition as described herein further comprising antibodies, electrolytes and soluble proteins. A composition as described herein may further comprise a cationic agent.

In another embodiment of the invention, the invention further provides a method for preparing a composition comprising a DNA dendrimer and a siRNA molecule, comprising the step of attaching the siRNA to the DNA dendrimer (a) via a disulfide bridging bond; (b) via the use of N-hydroxysuccinimide (NHS) ester dependent condensation reaction; (c) via the use of bifunctional cross linking reaction; or (d) via direct or indirect hybridization of the siRNA to DNA dendrimer sequence. In another embodiment of the invention, the invention further provides a method further comprising the step of attaching to the DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof.

The disclosure further provides a method of protecting a siRNA molecule against degradation, comprising the step of attaching (a) a siRNA molecule and (b) a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof to a DNA dendrimer, thereby protecting a siRNA molecule against degradation. The attachment of protein a FITC, a PEG molecule, biotin, a biotin derivative, or any combination thereof to the DNA dendrimer stabilized the DNA dendrimer and the siRNA molecule attached thereto. The attachment of protein, a FITC, a PEG molecule, biotin, a biotin derivative, fluorescein, or any combination thereof to the DNA dendrimer unexpectedly stabilizes the DNA dendrimer and the siRNA molecule attached thereto. The attachment of protein a FITC, a PEG molecule, biotin, a biotin derivative, fluorescein, or any combination thereof to the DNA dendrimer unexpectedly stabilizes the DNA dendrimer and the siRNA molecule attached thereto against serum degradation. The attachment of an antibody or a fragment thereof to the DNA dendrimer unexpectedly stabilizes the DNA dendrimer and the siRNA molecule attached thereto against serum degradation. The attachment of biotin to the DNA dendrimer unexpectedly stabilizes the DNA dendrimer and the siRNA molecule attached thereto against serum degradation. The attachment of PEG to the DNA dendrimer unexpectedly stabilizes the DNA dendrimer and the siRNA molecule attached thereto against serum degradation. The attachment of FITC to the DNA dendrimer unexpectedly stabilizes the DNA dendrimer and the siRNA molecule attached thereto against serum degradation. The attachment of fluorescein to the DNA dendrimer unexpectedly stabilizes the DNA dendrimer and the siRNA molecule attached thereto against serum degradation.

The disclosure, provided herein a method of protecting a siRNA molecule against degradation comprising the step of attaching to the DNA dendrimer a molecule comprising a DNA molecule, a RNA molecule, a protein, a peptide, a chemotherapeutic agent, an antiviral agent, an anti-inflammatory agent, a bacteriostatic agent, a psychoactive agent, a statin, a neuropathic agents, a hormone, an ACE inhibitor, an anti-clotting factor, an analgestic, an anti- angiogenic agent, a pro-angiogenic agent, a growth factor, a growth factor inhibitor, or any combination thereof.

The disclosure, provided herein a method of protecting a DNA dendrimer against degradation in a bodily fluid, comprising the step of attaching to a DNA dendrimer a protein, a peptide, an aptamer, a molecule containing a PEG moiety, biotin or a biotin derivative, fluorescein or a fluorescein derivative, a fluorescent dye, digoxigenin, cholesterol, primary amine, a hydrocarbon spacer of length 3 to 120 carbons, or any combination thereof, thereby protecting a DNA dendrimer against nuclease degradation in a bodily fluid.

The disclosure, provided herein a method of preparing a composition comprising a DNA dendrimer and a DNA molecule, a RNA molecule, a protein, a peptide, a chemotherapeutic agent, an antiviral agent, an anti-inflammatory agent, a bacteriostatic agent, a psychoactive agent, a statin, a neuropathic agents, a hormone, an ACE inhibitor, an anti-clotting factor, an analgestic, an anti- angiogenic agent, a pro-angiogenic agent, a growth factor, a growth factor inhibitor, or any combination thereof, comprising the step of attaching a siRNA to the DNA dendrimer (a) via a disulfide bridging bond; (b) via the use of NHS ester dependent condensation reaction; (c) via the use of heterobifunctional cross linking reaction; (d) via direct or indirect hybridization of the siRNA to DNA dendrimer sequence; or (e) via the use of polycationic compounds to bridge siRNA to the DNA dendrimer via charge-charge interactions.

It was unexpectedly found that the attachment of low molecular weight fluorescent dyes (less than 10,000 daltons) to the DNA dendrimer stabilized a composition comprising a DNA dendrimer or a composition comprising a DNA dendrimer and a siRNA molecule attached thereto. It was unexpectedly found that the attachment of low molecular weight fluorescent dyes (less than 10,000 daltons), comprising a fluorescent molecule attached to a linker or spacer, to the DNA dendrimer stabilized a composition comprising a DNA dendrimer or a composition comprising a DNA dendrimer and a siRNA molecule attached thereto. The attachment of low molecular weight fluorescent dyes (less than 10,000 daltons) comprising a fluorescent molecule attached to a linker or spacer to the DNA dendrimer stabilizes a composition comprising a DNA dendrimer or a composition comprising a DNA dendrimer and a siRNA molecule attached thereto. The attachment of cyanine dyes such as but not limited to Cy3 or Cy5 (Figure 8) to the DNA dendrimer unexpectedly stabilizes a composition comprising a DNA dendrimer or a composition comprising a DNA dendrimer and a siRNA molecule attached thereto. The attachment of Oyster dyes comprising an Oyster dye molecule attached to a linker or spacer, such as but not limited to Oyster 550 or Oyster 650 (Figure 9) to the DNA dendrimer unexpectedly stabilizes a composition comprising a DNA dendrimer or a composition comprising a DNA dendrimer and a siRNA molecule attached thereto. The attachment of Alexa Fluor dyes, comprising an Alexa Fluor molecule attached to a linker or spacer, such as but not limited to Alexa Fluor 350, Alexa Fluor 488, Alexa Fluor 532, Alexa Fluor 546, Alexa Fluor 555, Alexa Fluor 594, Alexa Fluor 633, Alexa Fluor 647, Alexa Fluor 655, Alexa Fluor 660, Alexa Fluor 680, or Alexa Fluor 700 to the DNA dendrimer unexpectedly stabilizes a composition comprising a DNA dendrimer or a composition comprising a DNA dendrimer and a siRNA molecule attached thereto. The attachment of fluorescein comprising an fluorescein molecule attached to a linker or spacer, and derivatives such as but not limited to FITC, 5/6-carboxyfluorescein succinimidyl ester, 5/6-FAM SE, or 5(6) fluorescein isothiocyanate mixed isomer, 5/6-FITC to the DNA dendrimer unexpectedly stabilizes a composition comprising a DNA dendrimer or a composition comprising a DNA dendrimer and a siRNA molecule attached thereto. The attachment of BODIPY 630/650 comprising a BODIPY molecule attached to a linker or spacer, to the DNA dendrimer unexpectedly stabilizes a composition comprising a DNA dendrimer or a composition comprising a DNA dendrimer and a siRNA molecule attached thereto.

The attachment of high molecular weight fluorescent dyes (greater than 50,000 daltons) such as but not limited to R-Phycoerythrin (conjugated to streptavidin), bound to biotin previously incorporated into the DNA dendrimer structure), B-Phycoerythrin (conjugated to streptavidin), bound to biotin previously incorporated into the DNA dendrimer structure), or Allophycocyanin (APC) (conjugated to streptavidin), bound to biotin previously incorporated into the DNA dendrimer structure) to the DNA dendrimer unexpectedly stabilizes a composition comprising a DNA dendrimer or a composition comprising a DNA dendrimer and a siRNA molecule attached thereto.

The attachment of non-fluorescent low molecular weight compounds such as but not limited to biotin and derivatives, NHS-biotin (for covalent binding to primary amines previously incorporated into the DNA dendrimer or oligonucleotide structure), Biotin-BB-CPG amidite (for synthesis of synthetic DNA oligonucleotides, Biotin-dT amidite amidite (for synthesis of synthetic DNA oligonucleotides, Biotin-BB-CPG amidite (for synthesis of synthetic DNA oligonucleotides), or digoxigenin derivatives to the DNA dendrimer unexpectedly stabilizes a composition comprising a DNA dendrimer or a composition comprising a DNA dendrimer and a siRNA molecule attached thereto.

The attachment of oligonucleitides, comprising Cholesterol TEG 5' / 3' amidite, propyl 3' spacer amidite, 12 carbon spacer amidite, 18 carbon spacer amidite, 6 carbon amino linker amidite, 12 carbon amino linker amidite, 7 carbon internal amino linker amidite, or dT internal amino linker amidite to the DNA dendrimer unexpectedly protects a composition comprising a DNA dendrimer or a composition comprising a DNA dendrimer and a siRNA molecule attached thereto from degradation.

In another embodiment of the disclosure, Cyanine, Oyster and Alexa Fluor dyes have been incorporated into the DNA dendrimer structure as: NHS-ester dye conjugates covalently bound to primary amines located either directly on the DNA dendrimer structure or on synthetic oligonucleotides subsequently hybridized and crosslinked to the DNA dendrimer structure. In another embodiment of the invention, DNA and/and RNA nucleotides incorporated during synthetic oligonucleotide synthesis (as labeled amidites) or via the use of RNA or DNA polymerases incorporating labeled RNA and/or DNA nucleotides (ribonucleotides and/or deoxyribonucleotides). In another embodiment of the disclosure, streptavidin conjugates that bind to biotin previously incorporated into the DNA dendrimer structure.

The disclosure further provides a method of protecting a DNA dendrimer against serum or body fluid degradation, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof, thereby protecting a DNA dendrimer against serum nuclease degradation. The disclosure further provides a method of protecting a DNA dendrimer against serum or body fluid degradation, comprising the step of attaching an antibody or a fragment thereof to a DNA dendrimer. The disclosure further provides a method of protecting a DNA dendrimer against serum or body fluid degradation, comprising the step of attaching biotin or a biotin derivative to a DNA dendrimer. The disclosure further provides a method of protecting a DNA dendrinier against serum degradation, comprising the step of attaching FITC to a DNA dendrimer. The disclosure further provides a method of protecting a DNA dendrimer against serum or body fluid degradation, comprising the step of attaching fluorescein or a fluorescein derivative to a DNA dendrimer.

The disclosure further provides a method of protecting a DNA dendrimer carrying a molecule comprising biological activity comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof, thereby protecting a DNA dendrimer carrying a molecule comprising biological activity against serum nuclease degradation. A molecule comprising biological activity may be an enzyme. A molecule comprising biological activity may be a therapeutic agent. A molecule comprising biological activity may be a chemotherapeutic agent. A molecule comprising biological activity may be a cytokine. A molecule comprising biological activity may be a chemokine. A molecule comprising biological activity may be a hormone. A molecule comprising biological activity may be a neurotransmitter. A molecule comprising biological activity may be a neurotransmitter precursor. A molecule comprising biological activity may be a neurotransmitter agonist. A molecule comprising biological activity may be a neurotransmitter antagonist. A molecule comprising biological activity may be a non-steroidial anitinflamatory agent. A molecule comprising biological activity may be a vitamin, a clotting factors, a chelator, is a peptide, a protein, an enzyme, a lipid.

The disclosure further provides a method of delivering a DNA dendrimer into a body fluid, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination, thereby delivering a DNA dendrimer into a serum. The disclosure further provides a method of delivering a DNA dendrimer into a body fluid, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination, thereby delivering a DNA dendrimer into a composition comprising serum or a body fluid. The disclosure further provides a method of delivering a DNA dendrimer into a serum or a body fluid, comprising the step of attaching to a DNA dendrimer an antibody or a fragment thereof, thereby delivering a DNA dendrimer into a composition comprising serum or a body fluid. The disclosure further provides a method of delivering a DNA dendrimer into a composition comprising serum or a body fluid, comprising the step of attaching to a DNA dendrimer biotin, thereby delivering a DNA dendrimer into a serum. The disclosure further provides a method of delivering a DNA dendrimer into a composition comprising serum or a body fluid, comprising the step of attaching to a DNA dendrimer a dye molecule, thereby delivering a DNA dendrimer a composition comprising serum or a body fluid. The disclosure further provides a method of delivering a DNA dendrimer into a serum, comprising the step of attaching to a DNA dendrimer a PEG, thereby delivering a DNA dendrimer a composition comprising serum or a body fluid.

The disclosure further provides a method of delivering a DNA dendrimer attached to a nucleic acid molecule into a serum, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof. The disclosure further provides a method of delivering a DNA dendrimer attached to a siRNA molecule into a serum, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination thereof.

Delivering may be in vitro mixing, or in vivo delivery. In vivo delivery of a composition as described herein or a DNA dendrimer modified with attachments as described is preformed by methods known to one of skill in the art such as but not limited to intravenous or intra-arterial injections.

The disclosure further provides a method of transfecting a cell with a siRNA molecule, comprising the step of contacting a cell with a composition comprising a DNA dendrimer and a siRNA molecule as described herein, thereby transfecting a cell with a siRNA molecule. The disclosure further provides a method of transfecting a cell with a siRNA molecule, comprising the step of contacting a cell with a composition comprising a DNA dendrimer, antibody or a fragment thereof, and a siRNA molecule as described herein, thereby transfecting a cell with a siRNA molecule. The disclosure further provides a method of transfecting a cell with a siRNA molecule, comprising the step of contacting a cell with a composition comprising a DNA dendrimer, biotin, and a siRNA molecule as described herein, thereby transfecting a cell with a siRNA molecule. The disclosure further provides a method of transfecting a cell with a siRNA molecule, comprising the step of contacting a cell with a composition comprising a DNA dendrimer, dye molecule, and a siRNA molecule as described herein, thereby transfecting a cell with a siRNA molecule.

A nucleic acid molecule may be atlached to the DNA dendrimer via a non covalent bond. A nucleic acid molecule may be attached to the DNA dendrimer via a covalent bond. In the invention, the nucleic acid molecule is siRNA. A nucleic acid molecule may be attached to the DNA dendrimer via hydrogen bonds such as Watson-Crick base pairing. A nucleic acid molecule may be attached to the DNA dendrimer via a disulfide bridging bond, via an NHS ester and an amine, via a heterobifunctional cross-linking bond. The term "attachment" as used herein may refer to a chemical bond.

The DNA strands may be covalently bonded in each layer of the dendrimer. The DNA dendrimer may comprise at least one Cap03 sequence located within the arm of the DNA dendrimer. The Cap03 sequence may comprise the following nucleic acid sequence: 5'-TCCACCTTAgAgTACAAACggAACACgAgAA-3' (SEQ ID NO: 8). The Cap03 sequence may be used for binding/attaching a molecule such as an antibody or a fragment thereof comprising a Cap03 anti-sense sequence, to the DNA dendrimer. The Cap03 anti-sense sequence may comprise the following nucleic acid sequence: 5'-TTCTCgTgTTCCgTTTgTACTCTAAggTggA-3' (SEQ ID NO: 9). A methods of preparing a composition as described herein may comprise the step of non-covalently binding a protein covalently conjugated to a DNA molecule comprising a sequence complementary to Cap03 sequence to the DNA dendrimer.

The nucleic acid molecule may comprise a binding moiety and the DNA dendrimer may comprise a binding partner wherein the binding partner non-covalently binds to the binding moiety.

A DNA dendrimer may be covalently cross linked. A DNA dendrimer may be a four layer DNA dendrimer. A DNA dendrimer may be a two layer DNA dendrimer. A DNA Dendrimer may be any dendrimer prepared from 3 or more strands of DNA.

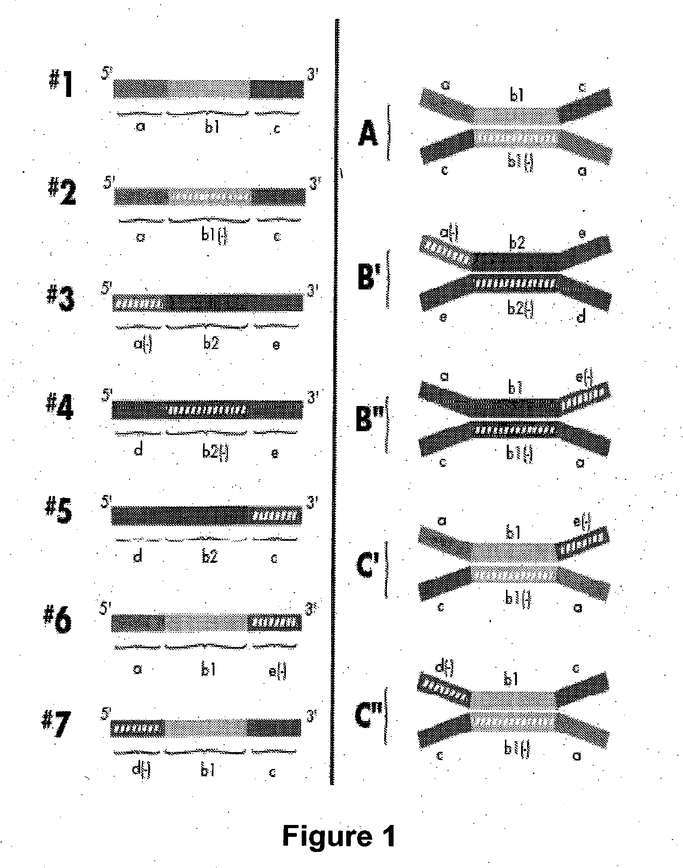

Methods are further provided for the manufacture and use of a DNA dendrimer containing a targeting antibody or a fragment thereof and a siRNA molecule non-covalently bound via a hybridization event (example 1). A DNA dendrimer as describer herein may be constructed from DNA monomers, each of which was made from two DNA strands that share a region of sequence complementarity located in the central portion of each strand. A DNA dendrimer may comprise a structure described as having a central doublestranded "waist" bordered by four single-stranded "arms". A DNA dedndrimer may comprise a waist-plus-arms structure which includes the basic DNA monomer. The single-stranded arms at the ends of each of the five monomer types may interact with one another in precise and specific ways. Base-pairing (hydrogen bonding) may allow directed assembly of the dendrimer through sequential addition of monomer layers (see Figure 1).

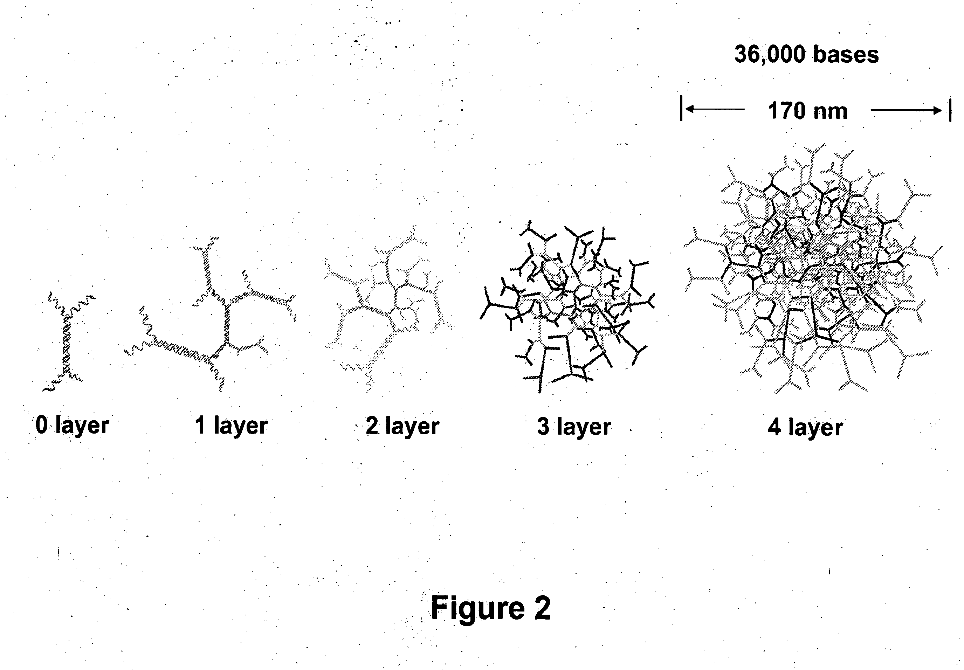

**A** DNA dendrimer as described herein may be assembled by a cross-linking process where the strands of DNA are covalently bonded to each other; thereby forming a completely covalent molecule impervious to denaturing conditions that otherwise would cause deformation of the dendrimer structure (see Figure 2).

A DNA dendrimer as described herein may be a two-layer dendrimer. A DNA dendrimer as described herein may comprise at least 60 biotin molecules, A DNA at least 80 biotin molecules, 100 biotin molecules, at least 120 biotin molecules, at least 150 biotin molecules, at least 180 biotin molecules, at least 200 biotin molecules, from 1 to 400 biotin molecules, from 60 to 360 biotin molecules, from 80 to 300 biotin molecules, from 100 to 250 biotin molecules, 150 to 250 biotin molecules, around 120 biotin molecules.

A DNA dendrimer as described herein may comprise at least 500 biotin molecules, at least 550 biotin molecules, at least 600 biotin molecules, at least 650 biotin molecules, at least 700 biotin molecules, A DNA dendrimer as described herein may comprise from 500 to 1500 biotin molecules, from 550 to 1400 biotin molecules, from 600 to 1000 biotin molecules, from 600 to 800 biotin molecules, from 700 to 800 biotin molecules, about 720 biotin molecules.

A DNA dendrimer as described herein may comprise a complementary capture oligonucleotide ligated to the 5' ends of available arms via a T4 DNA ligase dependent ligation reaction. A DNA dendrimer as described herein may comprise a complementary capture oligonucleotide (5-80 bases) ligated to the 5' ends of available arms via a T4 DNA ligase dependent ligation reaction. A DNA dendrimer as described herein may comprise a complementary capture oligonucleotided (10-60 bases) ligated to the 5' ends of available arms via a T4 DNA ligase dependent ligation reaction. A DNA dendrimer as described herein may comprise a complementary capture oligonucleotide (20-40 bases) ligated to the 5' ends of available arms via a T4 DNA ligase dependent ligation reaction. A DNA dendrimer as described herein may comprise a complementary capture oligonucleotide (30-50 bases) ligated to the 5' ends of available arms via a T4 DNA ligase dependent ligation reaction. A DNA dendrimer as described herein may comprise a complementary capture oligonucleotide (30-40 bases) ligated to the 5' ends of available arms via a T4 DNA ligase dependent ligation reaction.

A DNA dendrimer as described herein may comprise an antibody or a fragment thereof bound to the DNA dendrimers by first covalently conjugating a DNA oligonucleotide (complement to Cap03 sequence) to the antibody using cross-linking condensation conjugation chemistry, followed by hybridization of the antibody-bound oligonucleotide to a complementary sequence (Cap03) on the arms of the dendrimer. Sequences other than Cap03 can be used for attaching a molecule such as an antibody to the DNA dendrimer.

The DNA dendrimer as described herein comprises a siRNA molecule. The siRNA molecule may be chemically synthesized. The siRNA molecule may comprise a single stranded "sense" strand containing a 5 prime portion as RNA ribonucleotides, from 10-25 bases long, and an 3 prime portion as DNA deoxyribonucleotides, typically 0-40 bases long, which is designed to be complementary to the capture oligo ligated to the DNA dendrimer. A siRNA molecule may comprise a single stranded "sense" strand containing a 5 prime portion as RNA ribonucleotides, from 1-30 bases long, and an 3 prime portion as DNA deoxyribonucleotides, typically 1-50 bases long, which is designed to be complementary to the capture oligo ligated to the DNA dendrimer.

In another embodiment of the disclosure a siRNA as described herein comprises a single stranded "antisense" strand complementary to the "sense" strand, containing a portion of RNA ribonucleotides only and 5-40 bases long and 1-5 3-prime terminal deoxynucleotides. In another embodiment of the disclosure, a siRNA as described herein comprises a single stranded "antisense" strand complementary to the "sense" strand, containing a portion of RNA ribonucleotides only and 10-25 bases long and 1-3 3-prime terminal deoxynucleotides. In another embodiment of the disclosure, the siRNA constructs comprise 24-35 base extensions. In another embodiment of the disclosure, a siRNA as described herein comprises two strands combined in equimolar quantities to form stable hybrids between the "sense" and "antisense" strands, leaving the single stranded DNA portion of the "sense" strand available for hybridization to the DNA dendrimer's capture oligonucleotide.

In another embodiment of the invention, a composition comprising DNA dendrimer as described herein is used for *in-vitro* transfection. In an embodiment of the disclosure, a composition comprising DNA dendrimer as described herein is used for *in-vivo* transfection. In another embodiment of the disclosure, a composition comprising DNA dendrimer and a siRNA molecule as described herein is used for de-novo knockdown of a transcribed target gene. In another embodiment of the disclosure, a siRNA molecule is designed to target a transcribed target gene (mRNA).

In another embodiment of the disclosure, methods are provided herein for the manufacture and use of a DNA dendrimer containing a targeting antibody or a fragment thereof and a siRNA molecule where the siRNA molecules are non-covalently bound via the binding of biotinylated siRNA molecules to streptavidin, with subsequent binding to biotin on a DNA dendrimer. In another embodiment of the disclosure, provided herein methods for the manufacture and use of a DNA dendrimer containing a targeting antibody or a fragment thereof and a siRNA molecule where the siRNA molecules are non-covalently bound via the binding of biotinylated siRNA molecules to streptavidin, with subsequent binding to biotins on a DNA dendrimer.

In another embodiment of the disclosure, a composition as described herein comprising a DNA dendrimer bound with targeting antibody or a fragment thereof or any other molecule as provided is prepared as described above, except that biotin moieties are introduced onto the "arms" of the dendrimers through the hybridization and cross-linking of DNA or RNA oligonucleotides containing end labeled or internal biotins incorporated during the synthesis of the oligos. In another embodiment of the disclosure, a typical dendrimer biotin labeling reaction occurs prior to the binding of the antibody or a fragment thereof to the dendrimer, and during or after the ligation of the capture sequence.

In another embodiment of the disclosure, a biotinylated "sense" RNA of the invention forms an extremely strong non-covalent bond with 2-3 of the 4 available biotin binding valences available on the streptavidin molecule, leaving at least one free biotin binding streptavidin valence (on average) capable of binding a biotin moiety otherwise not associated with the "sense" RNA molecule.

In another embodiment of the disclosure, methods are provided herein for the manufacture of a DNA dendrimer comprising a targeting antibody or a fragment thereof and a siRNA molecule where the siRNA molecules are covalently bound via the use of disulfide bridging bonds. In another embodiment of the disclosure, a four layer DNA dendrimer or a two layer DNA dendrimer with antibody or a fragment thereof is synthesized according to in Example 1 or 2. In another embodiment of the disclosure, molecules designed to perform as siRNAs within the cell are chemically synthesized and comprise 1) a single stranded "sense" strand comprising all RNA ribonucleotides, typically 10-40 bases long, with 2-80 DNA nucleotides on the 3' end, and containing a sulhydryl (SH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another embodiment of the disclosure these two strands are combined in equimolar quantities to form stable hybrids between the "sense" and "antisense" strands, leaving the sulfhydryl moiety available for conjugation to another sulfhydryl moiety (to form a "S-S" disulfide bond) on a DNA oligonucleotide complementary to a capture sequence attached to the arms of the dendrimer.

In another embodiment of the disclosure, provided herein methods for the manufacture of a DNA dendrimer containing a targeting antibody or a fragment thereof and a siRNA molecule where the siRNA molecules are covalently bound via the use of NHS-ester dependent condensation chemistry. In another embodiment of the disclosure, a four layer DNA dendrimer or a two layer DNA dendrimer with antibody or a fragment thereof is synthesized as in Example 2 except that the biotins are replaced with primary amines. In another embodiment of the disclosure, molecules designed to perform as siRNAs within the cell are chemically synthesized and comprise 1) a single stranded "sense" strand comprising all RNA ribonucleotides, typically 5-50 bases long, with 2-80 DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another embodiment of the disclosure, the RNA strand containing the carboxyl is chemically modified using commercially available reagents such that the carboxyl was converted to an *N-*hydroxysuccinimide (NHS) ester, which in turn was reacted with a primary amine to form a covalent bond between the NHS ester and the amine. In another embodiment of the disclosure, a dendrimer labeled with primary amines is covalently bound with the "sense" strand of the siRNA, which when hybridized with the "antisense" RNA strand forms a functional siRNA duplex.

In another embodiment of the disclosure, methods are provided herein for the manufacture of a DNA dendrimer containing a targeting antibody or a fragment thereof and a siRNA molecule where the siRNA molecules are covalently bound via the use of heterobifunctional chemical cross-linker chemistry. In another embodiment of the disclosure, four layer DNA dendrimer or two layer DNA dendrimer with antibody or a fragment thereof is synthesized as in Example 2, except that the biotin molecules were replaced with primary amines. In another embodiment of the disclosure, molecules designed to perform as siRNAs within the cell were chemically synthesized and comprise 1) a single stranded "sense" strand comprising all RNA ribonucleotides, typically 5-50 bases long, with 2-80 DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another embodiment of the invention, the RNA strand containing the carboxyl is combined with the amine modified dendrimer in the presence of EDC (1-ethyl-3-[3-dimethylaminopropyl]carbodiimide), a heterobifunctional cross-linking reagent that formed a covalent bond between the carboxyl and amine moieties. In another embodiment of the disclosure, a dendrimer labeled with primary amines is covalently bound with the "sense" strand of the siRNA, which when hybridized with the "antisense" RNA strand formed a functional siRNA duplex.

In another embodiment of the disclosure, methods are provided herein for the manufacture of a DNA dendrimer containing a targeting antibody or a fragment thereof and a siRNA molecule where the siRNA molecules are covalently bound via the use of a homobifunctional chemical cross-linker chemistry. In another embodiment of the disclosure, four layer DNA dendrimer or two layer DNA dendrimer with antibody or a fragment thereof is synthesized as in Example 2, except that the biotins are replaced with primary amines. In another embodiment of the disclosure, molecules designed to perform as siRNAs within the cell are chemically synthesized and comprised 1) a single stranded "sense" strand comprising all RNA ribonucleotides, 5-40 bases long, with 2-80 DNA nucleotides on the 3' end, and containing a primary amine moiety attached during or after oligonucleotide synthesis on the 5' end of the molecule, and 2) a single stranded "antisense" strand complementary to the "sense" strand, containing a portion of RNA ribonucleotides only and 2-50 bases long, with two 3' terminal deoxynucleotides. In another embodiment of the disclosure, the RNA strand containing the amine is combined with the amine modified dendrimer in the presence of a homobifunctional cross-linker such as Sulfo-EGS [ethylene glycolbis(succinimidylsuccinate)], a reagent that forms a covalent bond between the amine moieties. In another embodiment of the disclosure, a DNA dendrimer labeled with primary amines is covalently bound with the "sense" strand of the siRNA, which was then hybridized with the "antisense" RNA strand to form a functional siRNA duplex.

In another embodiment of the disclosure, methods are provided herein for the manufacture of a DNA dendrimer containing a targeting antibody or a fragment thereof and a siRNA molecule and comparing the importance of biotin bound to the structure of the dendrimer as well as cations having multiple positive charges as counterions in transfection. In another embodiment of the disclosure, a two layer DNA dendrimer or a four layer DNA dendrimer with antibody or a fragment thereof is synthesized with up to 160 biotins populating the "arms" of the dendrimer, and a certain number of free "arms" on the dendrimer are available for binding of siRNA duplexes via a hybridization binding event (see Example 1 and 2). In another embodiment of the disclosure, a two layer DNA dendrimer or a four layer DNS dendrimer with antibody or a fragment thereof is synthesized with up to 140 biotins populating the "arms" of the dendrimer, and a certain number of free "arms" on the dendrimer are available for binding of siRNA duplexes via a hybridization binding event (see Example 1 and 2). In another embodiment of the invention, a two layer DNA dendrimer or a four layer DNA dendrimer with antibody or a fragment thereof is synthesized with up to 120 biotins populating the "arms" of the dendrimer, and a certain number of free "arms" on the dendrimer are available for binding of siRNA duplexes via a hybridization binding event (see Example 1 and 2).

In another embodiment of the disclosure, a composition of the invention comprises a siRNA construct comprising 10-40 base extension of the sense strand of the siRNA duplex and ICAM1 targeted dendrimers similar to those in Examples 1 and 2. In another embodiment of the disclosure, a composition of the invention comprising biotinylated dendrimer constructs and a siRNA molecule are more efficient for transfection than compositions lacking a biotin, especially in the presence of serum. In another embodiment of the disclosure, a composition of the invention comprising a cation is more efficient for transfection than compositions jacking a cation, especially in the presence of serum. In another embodiment of the disclosure, a composition of the invention comprising about 25mM cation is more efficient for transfection than compositions lacking a cation, especially in the presence of serum. In another embodiment of the disclosure, a composition of the invention comprising about 5-1000mM cation is more efficient for transfection than compositions lacking a cation, especially in the presence of serum. In another embodiment of the disclosure, the composition of the invention further comprises about 25mM of Mg, Ca, spermine, spermidine, or Mn and is more efficient for transfection than compositions lacking a cation, especially in the presence of serum. In another embodiment of the disclosure, the composition of the invention further comprises about 5-1000mM of Mg, Ca, spermine, spermidine, or Mn and is more efficient for transfection than compositions lacking a cation, especially in the presence of serum.

In another embodiment of the disclosure, methods are provided herein for knockdown of mRNA expression utilizing the compositions as described herein. In another embodiment of the disclosure, a composition of the invention comprising a protective group such as an antibody or a fragment thereof on both the two layer and four layer versions are synthesized with up to 180 biotins populating the "arms" of the two layer dendrimer, and up to 800 biotins populating the arms of the four layer dendrimer, with both types of dendrimers containing a certain number of free "arms" available for binding of siRNA duplexes via a hybridization binding event (see Examples 1 and 7).

In another embodiment of the disclosure, methods are provided herein for protecting a composition of the invention against nuclease dependent degradation of DNA dendrimers from exposure to protein nucleases in human and animal sera. In another embodiment of the disclosure, provided herein a method for protecting a composition of the invention from protein DNases degradation. In another embodiment of the disclosure, provided herein a method for protecting a composition of the invention from exogenous DNase degradation.

In another embodiment of the disclosure, methods are provided herein for combining a composition comprising a DNA dendrimer having hybridized siRNA molecules with commercial Lipofectamine transfection reagents. In another embodiment of the disclosure compositions comprising DNA dendrimers are combined with a transfection reagent for the cytoplasmic delivery of siRNA. In another embodiment of the disclosure, a composition is provided comprising a DNA dendrimer and Lipofectamine which improves the knockdown efficiency of the composition. In another embodiment of the disclosure, a composition comprising a DNA dendrimer and a liposomal transfection agent is provided which has an improved mRNA knockdown efficiency of siRNA molecules. In another embodiment of the disclosure, a composition is provided comprising a DNA dendrimer and other transfection agents familiar to one skilled in the art which has an improved mRNA knockdown efficiency of siRNA molecules.

In another embodiment of the disclosure, the compositions as described herein are provided to an individual *per se.* In another embodiment of the disclosure, the compositions as described herein are used as part of a diagnostic method. In another embodiment of the disclosure, the compositions as described herein are used as part of a therapeutic method. In one embodiment of the invention, the compositions as described herein are provided to the individual as part of a pharmaceutical composition where it is mixed with a pharmaceutically acceptable carrier.

A "pharmaceutical composition" refers to a preparation of one or more of the active ingredients described herein with other chemical components such as physiologically suitable carriers and excipients. The purpose of a pharmaceutical composition is to facilitate administration of a compound to an organism. '

"Active ingredient" refers to the compositions as described herein, which are accountable for the biological effect.

The present disclosure also provides combined preparations. "A combined preparation" defines especially a "kit of parts" in the sense that the combination partners as defined above can be dosed independently or by use of different fixed combinations with distinguished amounts of the combination partners i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. The combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

The phrases "physiologically acceptable carrier" and "pharmaceutically acceptable carrier", which may be interchangeably used, refer to a carrier or a diluent that does not cause significant irritation to an organism and does not abrogate the biological activity and properties of the administered compound. An adjuvant is included under these phrases. One of the ingredients included in the pharmaceutically acceptable carrier can be for example polyethylene glycol (PEG), a biocompatible polymer with a wide range of solubility in both organic and aqueous media (Mutter et al., 1979).

"Excipient" refers to an inert substance added to a pharmaceutical composition to further facilitate administration of an active ingredient. Excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition, which is incorporated herein by reference.

Suitable routes of administration, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

The preparation can be administered in a local rather than systemic manner, for example, via injection of the preparation directly into a specific region of a patient's body.

Various embodiments of dosage ranges are contemplated by this disclosure. The dosage of the composition of the present invention, may be in the range of 0.005-80 mg/day, 0.05-50 mg/day, 0.1-20 mg/day, 0.1-10 mg/day, 0.1-5 mg/day, 0.5-5 mg/day, 0.5-50 mg/day, 5-80 mg/day, 35-65 mg/day, 35-65 mg/day, 20-60 mg/day, 40-60 mg/day, 45-60 mg/day, 60-120 mg/day, 120-240 mg/day. 240-400 mg/day, 400-800 mg/day, 800-1600 mg/day, 15-25 mg/day, 5-10 mg/day, 55-65 mg/day.

In one embodiment of the disclosure, the dosage is 20 mg/day. In another embodiment of the disclosure, the dosage is 30 mg/day. In another embodiment of the disclosure, the dosage is 40 mg/day. In another embodiment of the disclosure, the dosage is 50 mg/day. In another embodiment of the disclosure, the dosage is 60 mg/day. In another embodiment of the disclosure, the dosage is 70 mg/day. In another embodiment of the disclosure, the dosage is 80 mg/day. In another embodiment of the disclosure, the dosage is 90 mg/day. In another embodiment of the disclosure, the dosage is 100 mg/day.

Peroral compositions, in some embodiments, comprise liquid solutions, emulsions, suspensions, and the like. In some embodiments, pharmaceutically-acceptable carriers suitable for preparation of such compositions are well known in the art. In some embodiments, liquid oral compositions comprise from about 0.012% to about 0.933% of the desired compound or compounds, or from about 0.033% to about 0.7%. The oral dosage form may comprise a predefined release profile.

In some embodiments, compositions for use in the methods of this invention comprise solutions or emulsions, which in some embodiments are aqueous solutions or emulsions comprising a safe and effective amount of the compounds of the present invention and optionally, other compounds, intended for topical intranasal administration. In some embodiments, compositions comprise from about 0.01% to about 10.0% w/v of a subject compound, more preferably from about 0.1% to about 2.0, which is used for systemic delivery of the compounds by the intranasal route.

In another embodiment of the disclosure, the pharmaceutical compositions are administered by intravenous, intra-arterial, or intramuscular injection of a liquid preparation. In some embodiments, liquid formulations include solutions, suspensions, dispersions, emulsions, oils and the like. In one embodiment of the disclosure, the pharmaceutical compositions are administered intravenously, and are thus formulated in a form suitable for intravenous administration. In another embodiment of the disclosure, the pharmaceutical compositions are administered intra-arterially, and are thus formulated in a form suitable for intra-arterial administration. In another embodiment of the disclosure, the pharmaceutical compositions are administered intramuscularly, and are thus formulated in a form suitable for intramuscular administration.

Further, in another embodiment of the disclosure, the pharmaceutical compositions are administered topically to body surfaces, and are thus formulated in a form suitable for topical administration. Suitable topical formulations include gels, ointments, creams, lotions, drops and the like. For topical administration, the compounds of the present invention are combined with an additional appropriate therapeutic agent or agents, prepared and applied as solutions, suspensions, or emulsions in a physiologically acceptable diluent with or without a pharmaceutical carrier.

In one embodiment of the disclosure, pharmaceutical compositions of the present invention are manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention are formulated in conventional manner using one or more physiologically acceptable carriers comprising excipients and auxiliaries, which facilitate processing of the active ingredients into preparations which, can be used pharmaceutically. In one embodiment of the invention, formulation is dependent upon the route of administration chosen.

Injectables of the invention may be formulated in aqueous solutions. In one embodiment of the disclosure, injectables of the invention are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment of the disclosure, the preparations described herein are formulated for parenteral administration, e.g., by bolus injection or continuous infusion. In some embodiments, formulations for injection are presented in unit dosage form, e.g., in ampoules or in multidose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances which increase the viscosity of the suspension, such as sodium carboxymethyl cellulose, sorbitol or dextran. In another embodiment of the disclosure, the suspension also contain suitable stabilizers or agents which increase the solubility of the active ingredients to allow for the preparation of highly concentrated solutions.

In another embodiment of the disclosure, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment of the disclosure, the pharmaceutical composition delivered in a controlled release system is formulated for intravenous infusion, implantable osmotic pump, transdermal patch, liposomes, or other modes of administration. In one embodiment of the disclosure, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment of the disclosure, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-1338 (1984). Other controlled release systems are discussed in the review by Langer (Science 249:1527-1533 (1990).

In some embodiments, the active ingredient is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In some embodiments, pharmaceutical compositions suitable for use in context of the present invention include compositions wherein the active ingredients are contained in an amount effective to achieve the intended purpose. In some embodiments, a therapeutically effective amount means an amount of active ingredients effective to prevent, alleviate or ameliorate symptoms of disease or prolong the survival of the subject being treated.

In one embodiment, determination of a therapeutically effective amount is well within the capability of those skilled in the art.

The compositions also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment of the invention, the pharmaceutically-acceptable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile, acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. In another embodiment of the invention, peroral liquid compositions also contain one or more components such as sweeteners, flavoring agents and colorants disclosed above.

The compositions also include incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of in vivo release, and rate of in vivo clearance.

Also comprehended by the disclosure are particulate compositions coated with polymers (e.g. poloxamers or poloxamines) and the compound coupled to antibodies directed against tissue-specific receptors, ligands or antigens or coupled to ligands of tissue-specific receptors.

In some embodiments, compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. In another embodiment, the modified compounds exhibit substantially longer half-lives in blood following intravenous injection than do the corresponding unmodified compounds. In one embodiment, modifications also increase the compound's solubility in aqueous solution, eliminate aggregation, enhance the physical and chemical stability of the compound, and greatly reduce the immunogenicity and reactivity of the compound. In another embodiment, the desired in vivo biological activity is achieved by the administration of such polymer-compound abducts less frequently or in lower doses than with the unmodified compound.

In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients-compositions as described herein can be determined by standard pharmaceutical procedures in vitro, in cell cultures or experimental animals. In one embodiment , the data obtained from these in vitro and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.1].

In one embodiment, depending on the severity and responsiveness of the condition to be treated, dosing can be of a single or a plurality of administrations, with course of treatment lasting from several days to several weeks or until cure is effected or diminution of the disease state is achieved.

In one embodiment, the amount of a composition to be administered will, of course, be dependent on the subject being treated, the severity of the affliction, the manner of administration, the judgment of the prescribing physician, etc.

In one embodiment, compositions including the preparation of the present invention formulated in a compatible pharmaceutical carrier are also be prepared, placed in an appropriate container, and labeled for treatment of an indicated condition.

In one embodiment, compositions of the present invention are presented in a pack or dispenser device, such as an FDA approved kit, which contain one or more unit dosage forms containing the active ingredient. In one embodiment of the invention, the pack, for example, comprise metal or plastic foil, such as a blister pack. In one embodiment of the invention, the pack or dispenser device is accompanied by instructions for administration. In one embodiment of the invention, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

It will be appreciated that the composition can be provided to the individual with additional active agents to achieve an improved therapeutic effect as compared to treatment with each agent by itself. In another embodiment of the disclosure, measures (e.g., dosing and selection of the complementary agent) are taken to adverse side effects which are associated with combination therapies.

Additional objects, advantages, and novel features of the present invention will become apparent to one ordinarily skilled in the art upon examination of the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference. Other general references are provided throughout this document.

### EXAMPLE 1

### Manufacture and use of a DNA dendrimer containing a targeting antibody or a fragment thereof and siRNA molecules non-covalently bound via a hybridization event

### MATERIALS AND METHODS:

DNA dendrimers are constructed from DNA monomers, each of which was made from two DNA strands that share a region of sequence complementarity located in the central portion of each strand. When the two strands anneal to form the monomer the resulting structure can be described as having a central double-stranded "waist" bordered by four single-stranded "arms". This waist-plus-arms structure comprises the basic DNA monomer. The single-stranded arms at the ends of each of the five monomer types are designed to interact with one another in precise and specific ways. Base-pairing between the arms of complementary monomers allowed directed assembly of the dendrimer through sequential addition of monomer layers (Figure 1). Assembly of each layer of the dendrimer included a cross-linking process where the strands of DNA were covalently bonded to each other, thereby forming a completely covalent molecule impervious to denaturing conditions that otherwise would cause deformation of the dendrimer structure (Figure 2). Further as described in Example 2 the dendrimers prepared for this example contained ∼120 biotin molecules (2-layer dendrimers) or -720 biotin molecules (4-layer dendrimers) attached to their outer surface. In addition, 38 base oligonucleotides that serve as complementary capture oligos were ligated to the 5' ends of available dendrimer arms via a simple T4 DNA ligase dependent ligation reaction, as follows:

**Table 1: The components that were added to a microfuge tube**

| | |
|---|---|
| two layer DNA dendrimer (500ng/uL) in 1X TE buffer | 5.4uL (2680ng) |
| a(-)LIG-BR7 Bridging oligo (14mer) (50ng/uL) | 2.7uL (134ng) |
| 10X Ligase buffer | 10.2uL |
| Nuclease free water | 81.7uL |
| Cap03 capture oligo (38mer) (50ng/uL) | 4.0uL (200ng) |
| T4 DNA Ligase (1 U/uL) | 10.0uL (10 units) |

The first four reactants were added together, heated to 65°C and cooled to room temperature. The 5^{th} and 6^{th} reactants were then added and incubated for 45 minutes. The ligation reaction was stopped by adding 2.8uL of 0.5M EDTA solution. Non-ligated oligonucleotide was removed via the use of a size exclusion spin column prepared using Sephacryl S400 (Pharmacia).

Antibodies were bound to DNA dendrimers by first covalently conjugating a DNA oligonucleotide (Complement to Cap03 sequence) to the antibody using previously described cross-linking condensation conjugation chemistry (prepared at Solulink Inc. (San Diego, CA), followed by hybridization of the antibody-bound oligonucleotide to a complementary sequence (Cap03) on the arms of the dendrimer. This hybridization comprises 31 base pairs and has a melting temperature of greater than 65°C. in a physiological salt solution, thereby providing a stable complex of dendrimer bound with antibody at physiological temperatures and conditions. A typical hybridization formulation included:

**Table 2: components that were added to a microfuge tube:**

| | |
|---|---|
| two layer DNA dendrimer with ligated Cap03 sequence (50ng/uL) | 50.0uL |
| 50% ethelyene glycol in PBS or equivalent (e.g. Superfreeze (Pierce)) | 25.0uL |
| 1X Phosphate Buffered Saline (PBS) | 57.0uL |
| 5M NaCl | 4.3uL |
| Oligo-Antibody Conjugate (anti-mouse, ICAM-1 antibody) (7.8ng/uL as oligo) | 13.7uL |

The above reactants were combined, gently mixed and incubated at 37°C for 30 minutes. This formulation is stable at 4°C for at least six months.

### SiRNA Design/Preparation

Molecules designed to perform as siRNAs within the cell were chemically synthesized and comprise 1) a single stranded "sense" strand containing a 5 prime portion as RNA ribonucleotides, typically 19 bases long, and an 3 prime portion as DNA deoxyribonucleotides, typically 0-33 bases long, which is designed to be complementary to the capture oligo ligated to the DNA dendrimer, and 2) a single stranded "antisense" strand complementary to the "sense" strand, containing a portion of RNA ribonucleotides only and typically 19 bases long and two 3 prime terminal deoxynucleotides. These two strands are combined in equimolar quantities to form stable hybrids between the "sense" and "antisense" strands, leaving the single stranded DNA portion of the "sense" strand available for hybridization to the DNA dendrimer's capture oligonucleotide.

For this experiment, the length of the deoxynucelotide extension (that links the siRNA to the dendrimer) versus the effectiveness of the siRNA when attached to 3 DNA molecules was studied. ICAM-1 on Hepa 1-6 cells was targeted and the Hepa 1-6 cells were grown in Delbecco's Modified Eagles Media (DMEM) with and without 10% fetal bovine serum (FBS) and used siRNAs designed to knockdown mouse ssb (La autoantigen) (Catalog number: S101433831, Qiagen GMBH, Hilden, Germany) mRNA verses a siRNA having no target (Catalog number: S1027310, Qiagen GMBH, Hilden,Germany).

### SiRNAs Tested:

### Condition #1: No Linker

### SSB SiRNAs: .

### Antisense strand:

RNA antisense strand : 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand: 5'- rUrUrA rArArG rUrCrU rGrUrU rGrUrC rArGrC rC dGdG -3' (SEQ ID NO: 10)

r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'-dTdT-3'

The complete sense strand is composed of the DNA sense strand bound to the 3' end of the RNA sense strand thus forming a RNA-DNA hybrid sense strand:
5'-rGrGrC rUrGrA rCrArA rCrArG rArCrU rUrUrA rA dTdT-3' (SEQ ID NO: 11)
r-refers to a ribonucleotide; d- refers to a deoxyribonucleotide;

### Negative Control siRNA:

### Antisense strand:

RNA antisense strand: 5'- ACGUGACACGUUCGGAGAA-3' (SEQ ID NO: 3)
DNA antisense strand 5'-dTdT-3'

The complete antisense strand is composed of the DNA antisense strand bound, to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUC UCCGAACGUGUCACGU -3' (SEQ ID NO: 4)
DNA sense strand: 5'-dTdT-3'

The complete sense strand is composed of the DNA sense strand bound to the 3' end of the RNA sense strand thus forming a RNA-DNA hybrid sense strand:
5'- rUrUrC rUrCrC rCrArA rCrGrU rGrUrC rArCrG rU dTdT-3' (SEQ ID NO: 13)
r-refers to a ribonucleotide; d- refers to a deoxyribonucleotide;

### Condition #2: SSB with 16 base RNA linker sequence:

### Antisense strand:

RNA antisense strand: 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'- TTCCGTTGACATCTCGTA -3' (SEQ ID NO: 5)

The complete sense strand is composed of the DNA sense strand bound to the 3' end of the RNA sense strand thus forming a RNA-DNA hybrid sense strand:
5'- rGrGrC rUrGrA rCrArA rCrArG rArCrU rUrUrA rA dTdT dCdCdGdTdT dGdAdC dAdTdCdTdC dGdTdA-3' (SEQ ID NO: 16)
r-refers to a ribonucleotide; d- refers to a deoxyribonucleotide;

### Negative Control (no mRNA target) with 16 base linker sequence:

### Antisense strand:

RNA antisense strand: 5'-ACGUGACACGUUCGGAGAA-3' (SEQ ID NO: 3)
DNA antisense strand 5'-dGdG-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT-3' (SEQ ID NO: 17).
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA ense strand: 5'- UUCUCCGAACGUGUCACGU-3' (SEQ ID NO: 4)
DNA sense strand: 5'- TTCCGTTGACATCTCGTA -3' (SEQ ID NO: 5)

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT dCdCdGdTdT dGdAdC dAdTdCdTdC dGdTdA -3' (SEQ ID NO: 18)
r-refers to a ribonucleotide; d- refers to a deoxyribonucleotide;

### Condition #3: SSB with 21 base DNA linker sequence:

### Antisense strand:

RNA antisense strand: 5'-UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'-TTCCGTTGACATCTCGTAGATTT-3' (SEQ ID NO: 6)

The complete sense strand is composed of the DNA sense strand bound to the 3' end of the RNA sense strand thus forming a RNA-DNA hybrid sense strand:
5' rGrGrC rUrGrA rCrArA rCrArG rArCrU rUrUrA rA dTdTdCdCdGdTdT dGdAdC dAdTdCdTdC dGdTdAdGdAdTdTdT 3' (Figure 12e, sense strand)
r-refers to a ribonucleotide; d- refers to a deoxyribonucleotide;

### Negative Control (no mRNA target) with 21 base linker sequence:

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT =3' (SEQ ID NO: 12)
r-refers to a ribonucleotide; d- refers to a deoxyribonucleotide; underlined nucleotide indicate a position within the oligonucleotide that would have a 2'-methoxy (also referred to as Omethyl) modifications.

### Sense strand:

RNA sense strand: 5'- UUCUCCGAACGUGUCACGU -3' (SEQ ID NO: 4)
DNA sense strand: 5'- TTCCGTTGACATCTCGTAGATTT -3' (SEQ ID NO: 6)

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT dCdCdGdTdT dGdAdC dAdTdCdTdC dGdTdAdGdAdTdTdT -3' (SEQ ID NO: 19)
r-refers to a ribonucleotide; d- refers to a deoxyribonucleotide;

### Condition #4: SSB with 26 base DNA linker sequence:

### Antisense strand:

RNA antisense strand: 5'- UUAAAGUCUGUUGUCAGCC -3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- GGCUGACAACAGACUUUAA-3' (SEQ ID NO: 2)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rGrGrC rUrGrA rCrArA rCrArG rArCrU rUrUrA rA dTdT dCdCdGdTdT dGdAdC dAdTdCdTdC dGdTdAdGdAdTdTdTdG dAdAdTdT -3' (SEQ ID NO: 15
r-refers to a ribonucleotide; d- refers to a deoxyribonucleotide;

### Negative Control (no mRNA target) with 26 base linker sequence:

### Antisense strand:

RNA antisense strand: 5'-ACGUGACACGUUCGGAGAA-3'(SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU -3' (SEQ ID NO: 4)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT dCdCdGdTdT dGdAdC dAdTdCdTdC dGdTdAdGdAdTdTdTdG dAdAdTdT -3' (SEQ ID NO: 14)
r-refers to a ribonucleotide; d- refers to a deoxyribonucleotide;

### SiRNA hybridization formulation:

"Sense" DNA/RNA molecule (50 microMolar) 25 µL
"Antisense" RNA molecule (50 microMolar) 25 µL

The sense and antisense siRNA oligonucleotides were combined in a microfuge per the formulation above. The oligo mixture was incubated at 80°C for 5 minutes then transferred to 37°C for 20 minutes to form the siRNA duplex. Prior to the preparation of a transfection mixture the hybridized siRNA was diluted by a factor of 25 fold in serum free media to a final concentration of 2 microMolar.

### Preparation of Transfection Mixtures

The following components were combined in a microfuge tube:
two layer DNA dendrimer with Antibody (10ng/µL) 12.0 µL
"SiRNA Duplex molecule (diluted to 2 microMolar) 3.0 µL
"Serum Free Media or PBS 105.0 µL

This mixture was incubated at 37°C for 20-30 minutes and then place at room temperature until use or at 4°C for longer term storage. For experiments in which other agents were added to the transfection mixture, the volume of the added component was subtracted from the amount of serum free media used in the transfection mixture. For example, transfection mixtures containing MgCl₂ were prepared by adding 7.5µl of 1M MgCl₂ and 97.5µl of serum free media in place of the 105µl of serum free media.

### Transfection Experiments:

The DNA dendrimer, containing the targeting antibody and the hybridized siRNA duplex, was introduced into wells in a tissue culture plate containing 2,000-10,000 live cells suitable as targets for *in-vitro* transfection and grown in the appropriate media containing 10% serum or in serum free media. These cells must contain certain features, including but not limited to 1) surface antigens suitable as binding targets for the dendrimer bound targeting antibody, 2) messenger RNA (mRNA) that will serve as an appropriate target for the siRNA antisense molecule bound to the dendrimer, 3) the ability to internalize the DNA dendrimer via an antibody mediated cell surface binding event, or other event(s) capable of initiating an endocytosis process. Typically, the above formulation was added to 100µL of tissue culture media in a 96 well plate at a volume ranging from 10-25% of the total volume of the well (10-25µl), although less or more may be required for the best effect. Function of the siRNA was measured directly by quantifying the amount of intact mRNA remaining in the cell after the addition of the dendrimer-siRNA complex using a qRT-PCR assay designed to detect and the targeted mRNA relative to an internal control mRNA (18s RNA and PPIB mRNA), or was measured indirectly by quantifying the amount of a protein remaining in the cell after the transfection of the siRNA and knockdown of expression of the mRNA target and associated protein(s).

As a control to confirm appropriate function of modified siRNAs, knockdown activity was confirmed for each modified siRNA using a commercially available transfection reagent, Lipofectamine 2000 (Invitrogen, Carlsbad, CA) according to the manufacture's recommendation and a final siRNA concentration of 10 nanoMolar. All knockdown determinations were relative to a Negative Control (no mRNA target) siRNA duplex containing the same structural modifications.

List of SiRNA modifications:
1. Wild-type siRNA Duplex consisting of both Control Unmodified unextended strands.
2. SiRNA Duplex consisting of the Sense Strand with a 16 base deoxynucleotide extension.
3. SiRNA Duplex consisting of the Sense Strand with a 21 base deoxynucleotide extension.

SiRNA Duplex consisting of the Sense Strand with a 26 base deoxynucleotide extension.

### Results

First the knockdown efficiency was measured for each of the siRNAs (10 nanomolar final concentration) independent of the DNA dendrimer delivery method using Lipofectamine 2000 by measuring the relative amount of SSB mRNA remaining compared to the appropriate negative control oligo. In all cases we observed between 70-95% knockdown efficiency. We then prepared DNA dendrimer hybridization mixtures having a 10nanoMolar final siRNA concentration and 0.2nanogram per microliter final DNA dendrimer concentration (∼2.5-3 nanoMolar as dendrimer bound siRNA for those capable of binding to dendrimer via the sequence extension) and compared knockdown efficiencies. In general, it was observed more significant knockdown efficiency in serum free media compared to serum containing media, most likely because of degradation of the siRNA. In both serum containing and serum free media comparisons, the siRNA constructs containing the longest (26 base) extension compared to the two shorter 3' deoxynucleotide extensions (21 and 16 bases, and no extension, respectively) performed best, yielding more knockdown of the target mRNA. In serum containing media we observed approximately 40% knockdown for the siRNA dulex with the 26 base 3' extension ranging down to little or no knockdown where the siRNA had no 3' extension of the sense strand. In serum free media, we observed approximately 65-70% knockdown for the 26 base 3' extended siRNA compared to 0-5%, 10-20%, and 30-35% knockdown for no 3' extension, 16 base extension, and 21 base extension, respectively.

Based on the results for the transfection comparing serum containing media to serum free media, the investigations continued to include siRNA modified with various chemical moieties in order to improve the stability of the siRNA in serum containing media. These modifications are listed above in the specification.

### EXAMPLE 2

### Manufacture and use or DNA dendrimer (containing a targeting antibody and siRNA molecules where the siRNA molecules are nort-covalently bound via the binding of biotinylated siRNA molecules to streptavidin, with subsequent binding to biotins on a DNA dendrimer

DNA dendrimers, bound with targeting antibodies are prepared as described above, except that biotin moieties are introduced onto the "arms" of the dendrimers through the hybridization and cross-linking of DNA or RNA oligonucleotides containing end labeled biotins incorporated during the synthesis of the oligos. A typical dendrimer biotin labeling reaction occurred prior to the binding of the antibody to the dendrimer, and during or after the ligation of the capture sequence, as follows:

The following components were added to a microfuge tube:
four layer DNA dendrimer with ligated Cap03 sequence (50ng/µL) 50.0µL
c(-) biotin oligo (500ng/µL) 2.6µL
a(-) biotin oligo (500ng/µL) 2.6µL
5M NaCl 4.0µL
2,4,8 trimethyl psoralen saturated in ethanol 7.0µL

The above reactants were added together, mixed well, and placed into a container of water at 65° and slow cooled to 42°C. Exposure to 300nm UV light for 10 minutes (X2) initiated a cross-linking event covalently binding the biotinylated oligos to the arms of the DNA dendrimer. Non-cross-linked oligonucleotides were removed via the use of a size exclusion spin column.

Biotin labeled oligonucleotides were sourced from commercial DNA oligonucleotide vendors. A variety of biotinylated phosphoramidites for synthesis of the biotinylated DNA oligonucleotides were used, including DNA synthesis reagents available from Glen Research Inc.and Trilink Biotechnology Inc., and include but are not limited to Biotin Phosphoramidite (Glen Research Cat # 10-1953-95), BiotinTEG Phosphoramidite (Glen Research Cat # 10-1955-95), Biotin-dT (Glen Research Cat # 10-1038-95), 5'-Biotin Phosphoramidite (Glen Research Cat # 10-5950-95), 5' Biotin (Trilink), Biotin Diol Linker (5' or Internal) (Trilink), 3' Biotin BB CPG (Trilink), and 5' Dual Biotin (Trilink). Other methods for the incorporation of biotin into nucleic acids using enzymatic and chemical synthesis will likely result in similar labeling efficiencies, including the incorporation of biotin into DNA using DNA polymerases and, biotinylated deoxyribonucleotides, the incorporation of biotin into RNA using DNA polymerases and biotinylated ribonucleotides, and the chemical incorporation of biotin into nucleic acids using technologies commercially available from Kreatech, Mirus Bio and other companies.

Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a biotin moiety (or biotin analog) attached during or after oligonucleotide synthesis on the 3' or 5' end of the molecule, and 2) a single stranded "antisense" strand complementary to the "sense" strand, containing a portion of RNA ribonucleotides only and typically 19 bases long, with two 3' terminal deoxyribonucleotides. These two strands were combined in equimolar quantities to form stable hybrids between the "sense" and "antisense" strands, leaving the biotin moiety available for binding to an avidin or streptavidin, molecule. The biotin-avidin binding formulation was:
SiRNA hybridization formulation:
   "Sense" DNA/RNA molecule (50 microMolar) with end biotin label 25 µL
   "Antisense" RNA molecule (50 microMolar) 25 µL

The sense and antisense siRNA oligonucleotides were combined in a microfuge per the formulation above. The oligo mixture was incubated at 80°C for 5 minutes then transferred to 37°C for 20 minutes to form the siRNA duplex.

The following components were added to a microfuge tube:
Hybridized duplex siRNA with end biotin label (50uM) 5.3µL
1X PBS 86.5µL
Streptavidin (1000ng/µL) 8.0µL
5M NaCl 0.2µL

The above reactants were combined, gently mixed and incubated at 37°C for 10 minutes.

In the above formulation, the biotinylated "sense" RNA formed an extremely strong non-covalent bond with 2-3 of the 4 available biotin binding valences available on the streptavidin molecule, leaving at least one free biotin binding streptavidin valence (on average) capable of binding a biotin moiety otherwise not associated with the "sense" RNA molecule.

The [biotinylated siRNA-streptavidin] complex was then mixed with the biotinylated dendrimer such that the remaining biotin binding valence(s) on the streptavidin bound to the biotin labels on the DNA dendrimer. This binding was accomplished by the following reaction:
four layer biotinylted DNA dendrimer with Antibody (10ng/µL) 50.0µL
"biotinylated siRNA-streptavidin" complex 5.3µL
1X PBS 3.7µL

The above reactants were combined, gently mixed and incubated at 37°C for 30 minutes.

The DNA dendrimer, containing the targeting antibody and the hybridized siRNA duplex, was introduced into wells in a tissue culture plate containing 2,000-10,000 live cells suitable as targets for *in-vitro* transfection. These cells contained certain features, including but not limited to 1) surface antigens suitable as binding targets for the dendrimer bound targeting antibody, 2) messenger RNA (mRNA) that served as an appropriate target for the siRNA antisense molecule bound to the dendrimer, 3) the ability to internalize the DNA dendrimer via an antibody mediated cell surface binding event, or other event(s) capable of initiating an endocytosis or internalization process. The above formulation was added to 100µL of tissue culture media in a 96 well plate at a volume ranging from 10-25% of the total volume of the well (10-25µl), although less or more may be required for the best effect. Function of the siRNA was measured directly by quantifying the amount of intact mRNA remaining in the cell after the addition of the dendrimer-siRNA complex, or was measured indirectly by quantifying the amount of a protein synthesized by the cell as a result of the degradation activity of the siRNA binding to a specific mRNA, resulting from the "knockdown" of expression of the mRNA and associated protein(s).

### EXAMPLE 3

### Manufacture of a DNA dendrimer containing a targeting antibody and siRNA molecules where the siRNA molecules are covalently bound via the use of disulfde bridging bonds

Four layer DNA dendrimer with Antibody (10ng/µL) is synthesized as in Example 1 or 2 above. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a sulhydryl (SH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. These two strands are combined in equimolar quantities to form stable hybrids between the "sense" and "antisense" strands, leaving the sulfhydryl moiety available for conjugation to another sulfhydryl moiety (to form a "S-S" disulfide bond) on a DNA oligonucleotide complementary to a capture sequence attached to the arms of the dendrimer. A typical sulfhydryl-sulfhydryl conjugation to a disulfide formulation would be:
The following components were added to a microfuge tube:
   "Sense" siRNA strand with end sulfhydryl (50uM) 10.0µL
   DNA oligo complementary to capture sequence with sulfhydryl (50uM) 9.0µL
   2M Dithiothreatol (DTT) aqueous 20.0µL
   Nuclease free water 1.0µL

The above reactants were combined, gently mixed and incubated at 65°C for 16 hours, or until most or all disulfide bonds were reduced to single sulfhydryl moieties. After incubation, the mixture was desalted and the buffer exchanged via the use of a commercial desalting column (Pierce, cat# 89891), yielding an equimolar solution of the "sense" RNA molecule and the DNA molecule, both containing S-H sulfhydryls resulting from the reduction of the disulfide formed after oligonucleotide synthesis. Stable disulfide bonds were formed between the DNA and RNA oligos either in the presence of mild oxidative conditions (exposure to air, oxygen or ozone) or via the addition of a mild oxidizing agent (hydrogen peroxide, 1-3%). On average, approximately 50% of the DNA and RNA disulfide complexes resulting from random formation of the disulfide bond will be of the appropriate DNA/RNA oligo combination, with 25% of the combinations comprising DNA/DNA and RNA/RNA disulfide complexes. The DNA/RNA complexes, which comprised a molecular weight and total length unique from the DNA/DNA and RNA/RNA complexes, were purified on an HPLC or via PAGE electrophoresis processes. The resulting DNA/RNA complex containing the disulfide bond between the DNA and RNA oligos was then hybridized to the DNA dendrimer as discussed in Example 1.

### EXAMPLE 4

### Manufacture of a DNA dendrimer containing a targeting antibody and siRNA molecules where the siRNA molecules are covalently bound via the use of NHS-ester dependent condensation chemistry

Four layer DNA dendrimer with Antibody (10ng/uL) was synthesized as in Example 2 above, except that the biotins were replaced with primary amines. Molecules designed to perform as siRNAs within the cell were chemically synthesized and comprised 1) a single stranded "sense" strand comprising all RNA ribonucleotides, typically 19-23 bases long, with two or more -DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. Typically, the RNA strand containing the carboxyl was chemically modified using commercially available reagents such that the carboxyl was converted to an *N-*hydroxysuccinimide (NHS) ester, which in turn was reacted with a primary amine to form a covalent bond between the NHS ester and the amine. A dendrimer labeled with primary amines can thus be covalently bound with the "sense" strand of the siRNA, which when hybridized with the "antisense" RNA strand forms a functional siRNA duplex.

The conversion of the carboxyl to the NHS was performed as below:
The following components were added to a microfuge tube:
   "Sense" siRNA strand with end carboxyl (500ng/µL) in ultrapure water 1000µL
   EDC (1-ethyl-3-[3-dimethylaminopropy]]carbodiimide) 0.4mg
   Sulfo-NHS reagent (Pierce cat# 24510) 1.1mg

After incubation for 15 minutes at room temperature (15-30°C), the mixture was desalted and the buffer exchanged via the use of a commercial desalting column (Pierce, cat# 89891). The exchange buffer was 1X PBS pH 7.4 (containing no amines).

Next, the "sense" RNA molecule now containing an activated NHS ester was added to a DNA dendrimer containing primary amines (in water or 1X PBS buffer containing no amines). This reaction was allowed to proceed for 2 hours at room temperature. After the incubation, 1M Tris-HCl was added to a final concentration of 50mM, which "quenched" the reaction of the NHS-esters. 5M NaCl was added to a final concentration of 100mM. The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column as previously described.

### EXAMPLE 5

### Manufacture of a DNA dendrimer containing a targeting antibody and siRNA molecules where the siRNA molecules are covalently bound via the use of a heterobifunctional chemical cross-linker chemistry

Four layer DNA dendrimer with Antibody (10ng/µL) was synthesized as in Example 2 above, except that the biotin molecules were replaced with primary amines. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. The RNA strand containing the carboxyl was combined with the amine modified dendrimer in the presence of EDC (1-ethyl-3-[3-dimethylaminopropyl]carbodiimide), a heterobifunctional cross-linking reagent that formed a covalent bond between the carboxyl and amine moieties. Thus, a dendrimer labeled with primary amines was covalently bound with the "sense" strand of the siRNA, which when hybridized with the "antisense" RNA strand formed a functional siRNA duplex.

The crosslinking of the "sense" RNA strand containing a carboxyl to the primary amines pre-bound to the dendrimer was performed as below:
The following components were added to a microfuge tube:
   "Sense" siRNA strand with end carboxyl (500ng/µL) in ultrapure water 100.0µL four layer amine dendrimer with capture sequence (500ng/µL) in 1X PBS 100.0µL
   EDC (1-ethyl-3-[3-dimethylaminopropyl]carbodiimide) 10.0mg

The above reaction was incubated for 2 hours at room temperature (15-30°C). After incubation, the mixture was desalted and the buffer exchanged via the use of a commercial desalting column (Pierce, cat# 89891). The exchange buffer used was 1X PBS pH 7.4 (containing no amines).

The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column. Antibody was also bound to the dendrimer as described in Examples 1 and 2.

### EXAMPLE 6

### Manufacture of a DNA dendrimer containing a targeting antibody and siRNA molecules where the siRNA molecules are covalently bound via the use of a homobifunctional chemical cross-linker chemistry

Four layer DNA dendrimer with Antibody (10ng/µL) was synthesized as in Example 2 above, except that the biotins are replaced with primary amines. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a primary amine moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. The RNA strand containing the amine was combined with the amine modified dendrimer in the presence of a homobifunctional cross-linker such as Sulfo-EGS [ethylene glycolbis(succinimidylsuccinate)] (Pierce cat# 21566), a reagent that forms a covalent bond between the amine moieties. Thus, a dendrimer labeled with primary amines was covalently bound with the "sense" strand of the siRNA, which was then hybridized with the "antisense" RNA strand to form a functional siRNA duplex.

The crosslinking, of the "sense" RNA strand containing a primary amine to the primary amines pre-bound to the dendrimer was performed as below.

The following components were added to a microfuge tube:
"Sense" siRNA strand with end amine (500ng/µL) in ultrapure water 100.0µL
four layer amine dendrimer with capture sequence (500ng/µL) in 1X PBS 100.0µL
Sulfo-EGS [ethylene glycolbis(succinimidylsuccinate)] 20.0mg

The above reaction was incubated for 2 hours at room temperature (15-30°C). After incubation, the mixture was desalted and the buffer exchanged via the use of a commercial desalting column (Pierce, cat# 89891). The exchange buffer used was 1X PBS pH 7.4 (containing no amines).

The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column. Antibody was also bound to the dendrimer as described in Examples 1 and 2.

### EXAMPLE 7

### Manufacture of a DNA dendrimer containing a targeting antibody and siRNA molecules and comparing the importance of biotin bound to the structure of the dendrimer as well as cations having multiply positive charges as counterions in transfection

A two layer DNA dendrimer with antibody was synthesized with up to 120 biotins populating the "arms" of the dendrimer, and a certain number of free "arms" on the dendrimer are available for binding of siRNA duplexes via a hybridization binding event (see Example 1 and 2).

Using a siRNA construct consisting of a 26 base extension of the sense strand of the siRNA duplex (best result in Example 1) and ICAM1 targeted dendrimers similar to those in Examples 1 and 2, we examined the importance of biotin on the dendrimer delivery platform. For this experiment we compared dendrimers prepared with and without biotin on the outer surface. The same methods for dendrimer and a siRNA molecule preparation as well as combining of the two and used in transfection knockdown studies were used as described in Example 1. Further in combination with biotinylated dendrimer constructs in siRNA knockdown assays we compared targeted dendrimer siRNA transfection efficiency with or without Mg 2+, Ca 2+, and Mn2+ in the assay as part of the initial hybridization of siRNA dendrimer construct by adding 1M of the appropriate cation salt solution to a final concentration or 125 mM and subsequently using 25ul of this mixture combined with plated cells in 100µl of serum or serum free media.

### Results

We observed that dendrimer constructs prepared with biotin on the outer surface demonstrated a 65-80% knockdown, while dendrimers without biotin only produced approximately 35-40% knockdown when tested in serum free media (as described in Example 1). In serum containing media a similar ratio of performance was observed between dendrimer with biotin verses without biotin, the siRNA knockdown activity being about 25-50% of that observed in serum free media for dendrimers without biotin compared to their biotinylated counterpart. Similarly, we observed that Mg, Ca, and Mn at a final concentration of 25mM yielded more reproducible and efficient knockdown compared to transfections in which cation was omitted. We also expect that other cations such as spermine and spermidine will demonstrate similar benefits when using dendrimers in these types of experiments.

### EXAMPLE 8

### Use of 2 and four layer dendrimers for siRNA knockdown of mRNA expression

DNA dendrimers with antibody on both the two layer and four layer versions are synthesized with up to 120 biotins populating the "arms" of the two layer dendrimer, and up to 720 biotins populating the arms of the four layer dendrimer, with both types of dendrimers containing a certain number of free "arms" available for binding of siRNA duplexes via a hybridization binding event (see Examples 1 and 7). The same methods for siRNA preparation, combining of dendrimer with siRNA, and transfection knockdown studies were used as described in Example 1. 2-payer versus 4-layer dendrimer results were compared. The final siRNA and dendrimer concentration (by mass) were used for both 2 and four layer constructs to insure that equal amounts of siRNA molecules were bound to dendrimer molecules.

### Results

The results indicate when using equal input mass of each dendrimer type at the same siRNA final concentration that 2-layer dendrimers produce 1.5-2 fold greater knockdown than do 4-layer dendrimer even though each 2-layer dendrimer has 1/9^{th} the amount of siRNA molecules per dendrimer.

### EXAMPLE 9

### Protection from nuclease dependent degradation of DNA dendrimers from exposure to protein nucleases in human and animal sera

Unmodified DNA dendrimers are subject to nuclease dependent degradation when exposed to solutions containing protein DNases. Therefore, it was logical to presume that DNA dendrimers, either unmodified or modified with various hapten, flourescent, amine or other labels and targeting antibodies, would quickly degrade when introduced into an *in-vitro* or *in-vivo* environment containing fluids derived from animal sources (e.g serum). This assumption historically precluded our use of DNA dendrimers for *in-vitro* cellular assays and any *in*-*vivo* applications until very recently, when it was unexpectedly observed that DNA dendrimers (prepared according to the prior examples 1-8) showed significant resistance to nuclease dependent degradation for at least 960 minutes (16 hours). The experiment was performed as follows:

Experiment 1: incubation of unmodified and modified DNA dendrimer with 0% and 75% human serum, with and without additional exogenous DNase added.

### Conditions.

Tubes 1-4 contained unmodified four layer DNA dendrimers, with the following additives:
Tube 1: In PBS only.
Tube 2: In 75% fresh human serum.
Tube 3: In PBS with 1U of exogenous DNase.
Tube 4: In 75% fresh human serum with 1U of exogenous DNase.

Tubes 5-8 contained modified four layer DNA dendrimers, containing ∼960 FITC dyes per dendrimer (on average) and 15-25 anti-ICAM1 mouse monoclonal antibodies (attached as described in example 1), with the following additives:
Tube 5: In PBS only.
Tube 6: In 75%. fresh human serum.
Tube 7: In PBS with 1U of exogenous DNase.
Tube 8: In 75% fresh human serum with 1U of exogenous DNase.

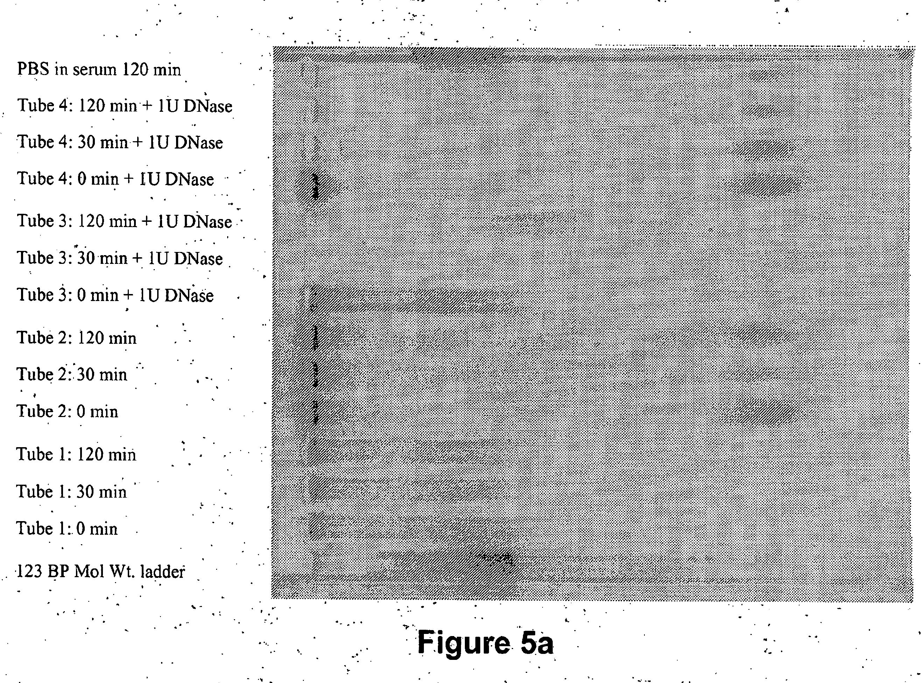

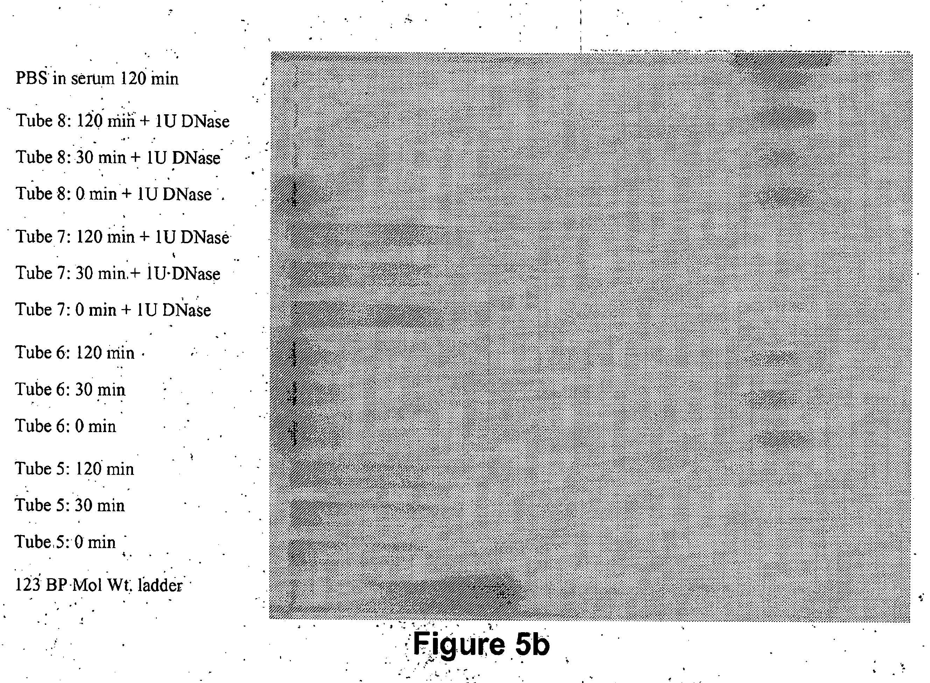

Each tube was incubated at 37°C for 0, 30 and 120 minutes. Samples were removed from each tube at each time-point and EDTA was added immediately to the removed samples to a final concentration of 50mM in order to stop the nuclease degradation via simple chelation of critical cations required for nuclease activation. Degradation of the dendrimers was observed after each samples was electrophoresed for 3 hours on an 0.8% agarose gel at 75 volts (figures 5a and 5b).

The gel results clearly indicated the following results:
Tube 1: No degradation observed after 120 minutes.
Tube 2: No degradation observed after 120 minutes.
Tube 3: Obvious degradation observed at the 30 minute timepoint.
Tube 4: Obvious degradation observed at the 30 minute timepoint.
Tube 5: No degradation observed after 120 minutes.
Tube 6: No degradation observed after 120 minutes.
Tube 7: No degradation observed after 120 minutes.
Tube 8: Obvious degradation observed at the 30 minute timepoint.

### Results

Degradation of unmodified DNA dendrimers occurred only in the presence of 1U of exogenous DNase in both the PBS and 75% serum conditions. Degradation of the modified DNA dendrimers occurred only in the presence of 1U of exogenous DNase in the 75% serum condition only.

*Conclusion:* both the unmodified and modified DNA dendrimers demonstrated unexpected resistance to nuclease degradation in human serum, although the modified DNA dendrimer further showed resistance to exogenous DNase in the PBS buffer but not in the human serum. This was an unexpected result, as prior results indicated that unmodified DNA dendrimers were severely degraded after a relatively brief exposure (30 minutes) to animal serum.

Experiment 2: incubation of unmodified and modified DNA dendrimer with 0% and 75% human serum for intervals up to 960 minutes (16 hours).

### Conditions

Tubes 1-4 contained unmodified four layer DNA dendrimers, with the following additives:
Tube 1: PBS only.
Tube 2: 75% fresh human serum.

Tubes 3-4 contained modified four layer DNA dendrimers, containing -960 FITC dyes per dendrimer (on average) and 15-25 anti-ICAM1 mouse monoclonal antibodies (attached as described in example 1), with the following additives:
Tube 3: PBS only.
Tube 4: 75% fresh human serum.

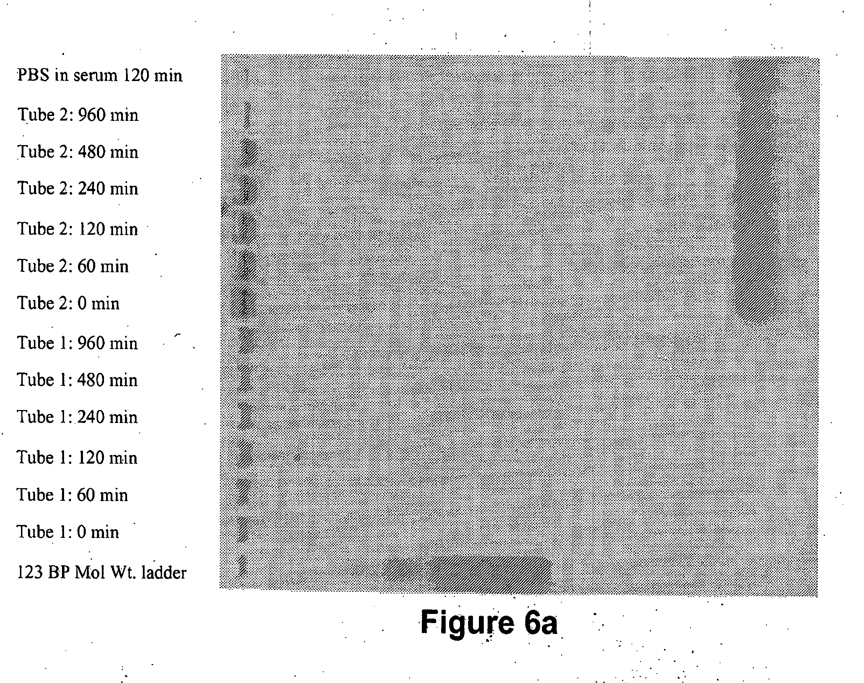

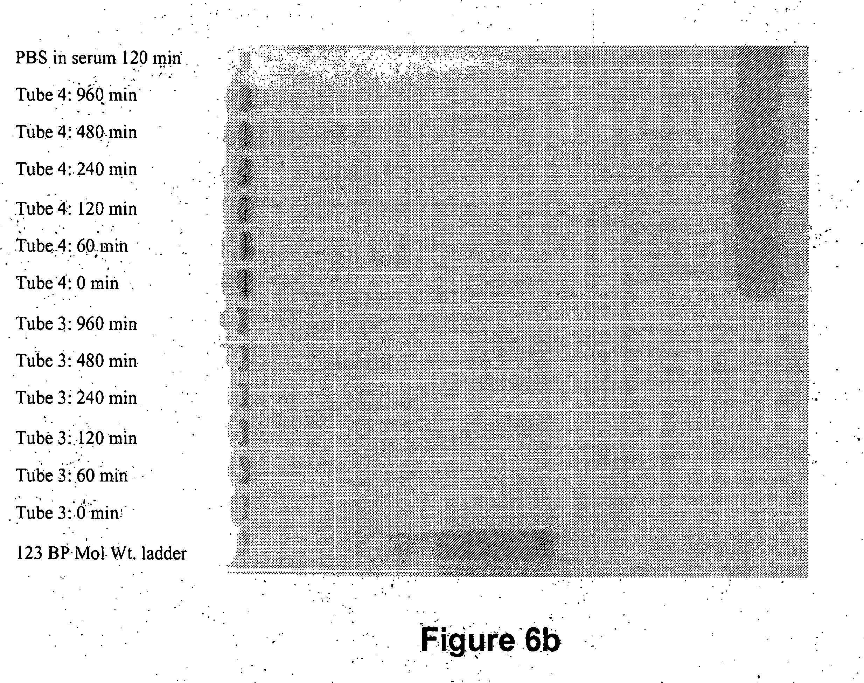

Each tube was incubated at 37°C for 0, 60 and 120, 240, 480 and 960 minutes. Samples were removed from each tube at each time-point and 50mM EDTA was added immediately to the removed samples to stop the nuclease degradation via simple chelation of critical cations required for nuclease activation. Degradation of the dendrimers was observed after each samples was electrophoresed for 3 hours on an 0.8% agarose gel at 75 volts (figures 6a and 6b The gel results clearly indicated the following results:
Tube 1: No degradation observed after 960 minutes.
Tube 2: No degradation observed after 480 minutes, with >80% degradation observed at the 960 minute time point.
Tube 3: No degradation observed after 960 minutes.
Tube 4: No degradation observed after 960 minutes.

### Results

Degradation of unmodified DNA dendrimers occurred only in the presence of 75% human serum sometime after the 480 minute time-point. Degradation of the modified DNA dendrimers was not observed at any timepoint for either the PBS or 75% serum conditions.

*Conclusion:* both the unmodified and modified DNA dendrimers demonstrated unexpected resistance to nuclease degradation in human serum, although the modified DNA dendrimer further showed somewhat more resistance to serum based nuclease degradation when compared to the unmodified DNA dendrimer. This was also an unexpected result, demonstrating the stability of modified DNA dendrimers for at 16 hours in 75% fresh human serum at 37°C. We believe that the addition of the FITC and antibody modifications to the DNA dendrimer provides some additional level of protection otherwise not expected from prior experiences with DNA molecules otherwise not chemically modified to resist nucleases in human serum (e.g. phosphothiorate chemistry modification, which was not used for these DNA dendrimers).

### EXAMPLE 10

### Combination of DNA dendrimers having hybridized siRNA molecules with commercial Lipofectamine transfection reagents

The goal of this experiment was to determine if DNA dendrimers independent of a targeting antibody and having a siRNA molecule attached via hybridization can be successfully combined with another transfection reagent for the cytoplasmic delivery of siRNA as measured by mRNA knockdown.

DNA dendrimers were prepared as outlined in example 1 and as previously disclosed (see patents 5,175,270, 5,484,904, 5,487,973, 6,110,687, and 6,274,723). Briefly, a DNA dendrimer was constructed from DNA monomers, each of which was made from two DNA strands that share a region of sequence complementarity located in the central portion of each strand. When the two strands anneal to form the monomer the resulting structure can be described as having a central double-stranded "waist" bordered by four single-stranded "arms". This waist-plus-arms structure comprises the basic DNA monomer. The single-stranded arms at the ends of each of the five monomer types are designed to interact with one another in precise and specific ways. Base-pairing between the arms of complementary monomers allowed directed assembly of the dendrimer through sequential addition of monomer layers (figure 1). Assembly of each layer of the dendrimer included a cross-linking process where the strands of DNA were covalently bonded to each other, thereby forming a completely covalent molecule impervious to denaturing conditions that otherwise would cause deformation of the dendrimer structure (figure 2). The dendrimers prepared for this example contained either no biotin or up to -720 biotin molecules (4-layer dendrimers) attached to their outer surface. For some conditions tested antibodies were combined with dendrimers by first attaching 38 base oligonucleotides that serve as complementary capture oligos were ligated to the 5' ends of available dendrimer arms via a simple T4 DNA ligase dependent ligation reaction, as follows:

The following components were added to a microfuge tube:
two layer DNA dendrimer (500ng/µL) in 1X TE buffer 5.4µL (2680ng)
a(-)LIG-BR7 Bridging oligo (14mer) (50ng/µL) 2.7µL (134ng)
10X Ligase buffer 10.2µL
   Nuclease free water 81.7µL
Cap03 capture oligo (38mer) (50ng/µL) 4.0µL (200ng)
T4 DNA Ligase (1 U/µL)10.0µL (10 units)

The first four reactants were added together, heated to 65°C and cooled to room temperature. The 5^{th} and 6^{th} reactants were then added and incubated for 45 minutes. The ligation reaction was stopped by adding 2.8µL of 0.5M EDTA solution. Non-ligated oligonucleotide was removed via the use of a size exclusion spin column prepared using Sephacryl S400 (Pharmacia).

Antibodies were bound to DNA dendrimers by first covalently conjugating a DNA oligonucleotide (Complement to Cap03 sequence) to the antibody using previously described cross-linking condensation conjugation chemistry (prepared at Solulink Inc. (San Diego, CA), followed by hybridization of the antibody-bound oligonucleotide to a complementary sequence (Cap03) on the arms of the dendrimer. This hybridization comprises 31 base pairs and has a melting temperature of greater than 65°C. in a physiological salt solution, thereby providing a stable complex of dendrimer bound with antibody at physiological temperatures and conditions.

A typical hybridization formulation:
two layer DNA dendrimer with ligated Cap03 sequence (50ng/µL) 50.0µL
50% ethelyene glycol in PBS or equivalent (e.g. Superfreeze (Pierce)) 25.0µL
1X Phosphate Buffered Saline (PBS) 57.0µL
5M NaCl 4.3µL
Oligo-Antibody Conjugate (anti-mouse ICAM-1 antibody) (7.8ng/µL as oligo) 13.7µL

The above reactants were combined, gently mixed and incubated at 37°C for 30 minutes. This formulation is stable at 4°C for at least six months.

### SiRNA Design/Preparation

Molecules designed to perform as siRNAs within the cell are chemically synthesized and comprise 1) a single stranded "sense" strand containing a 5 prime portion as RNA ribonucleotides, typically 19 bases long, and an 3 prime portion as DNA deoxyribonucleotides, typically 0-33 bases long, which is designed to be complementary to the capture oligo ligated to the DNA dendrimer, and 2) a single stranded "antisense" strand complementary to the "sense" strand, containing a portion of RNA ribonucleotides only and typically 19 bases long and two 3 prime terminal deoxynucleotides. These two strands are combined in equimolar quantities to form stable hybrids between the "sense" and "antisense" strands, leaving the single stranded DNA portion of the "sense" strand available for hybridization to the DNA dendrimer's capture oligonucleotide.

For this experiment we studied siRNA molecules either directly attached to dendrimers via hybridization of a 26 base long linker sequence or unattached. In some cases, antibodies were pre-attached to dendrimer as outlined by the conditions listed below.

For antibody containing dendrimers we attached anti-ICAM-1 because this antibody is observed on the cell surface of Hepa 1-6 cells grown in Delbecco's Modified Eagles Media (DMEM) with and without 10% fetal bovine serum (FBS). For all cases we used siRNAs designed to knockdown mouse ssb (La autoantigen) mRNA verses a siRNA having no target (Qiagen).

### SiRNAs Tested:

### siRNAs without dendrimer attachment sequence:

### SSB-SiRNAs (SSB unmod):

### Antisense strand:

RNA antisense strand: 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArGrUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'-dTdT-3'

The complete sense strand is composed of the DNA sense strand bound to the 3' end of the RNA sense strand thus forming a RNA-DNA hybrid sense strand:
5'-rGrGrC rUrGrA rCrArA rCrArG rArCrU rUrUrA rA dTdT-3' (SEQ ID NO: 11)
r-refers to a ribonucleotide; d- refers to a deoxyribonucleotide;

### Negative Control SiRNA (Neg unmod):

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (a]so referred to as Omethyl) modification.

### Sense strand:

RNA sense strand: 5'- UUC UCCGAACGUGUCACGU -3' (SEQ ID NO: 4),
DNA sense strand: 5'-dTdT-3'

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT -3' (SEQ ID NO: 13).
r-refers to a ribonucleotide; d- refers to a deoxyribonucleotide;

### SSB with 26 base DNA linker sequence (SSB+26):

### Antisense strand:

RNA antisense strand: 5'- UUAAAGUCUGUUGUCAGCC -3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position withih the oligonucleotide that would have a 2'- methoxy (also referred to as Omethyl) modification.

### Sense strand:

RNA sense strand: 5'- GGCUGACAACAGACUUUAA-3' (SEQ ID NO: 2)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rGrGrC rUrGrA rCrArA rCrArG rArCrU rUrUrA rA dTdT dCdCdGdTdT dGdAdC dAdTdCdTdC dGdTdAdGdAdTdTdTdG dAdAdTdT -3' (SEQ ID NO: 14)
r-refers to a ribonucleotide; d- refers to a deoxyribonucleotide;

### Negative Control (no mRNA target) with 26 base linker sequence:

### Antisense strand:

RNA antisense strand: 5'-ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU -3' (SEQ ID NO: 4)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT dCdCdGdTdT dGdAdC dAdTdCdTdC dGdTdAdGdAdTdTdTdG dAdAdTdT -3' (SEQ ID NO: 15)
r-refers to a ribonucleotide; d- refers to a deoxyribonucleotide;

SiRNA hybridization formulation:
"Sense" DNA/RNA molecule (50 microMolar) 25 µL
"Antisense" RNA molecule (50 microMolar) 25 *L

The sense and antisense siRNA oligonucleotides were combined in a microfuge tube per the formulation above. The oligo mixture was incubated at 80°C for 5 minutes then transferred to 37°C for 20 minutes to form the siRNA duplex. Prior to the preparation of a transfection mixture the hybridized siRNA was diluted by a factor of 25 fold in serum free media to a final concentration of 2 microMolar.

### Preparation of Transfection Mixtures

Lipofectamine 2000 (Invitrogen, Carlsbad, CA) was used according to the manufacturer and first diluted to a 2 times concentrate in serum free media to prepare a 2x Lipofectamine solution.

SiRNA only Lipofectamine complexes:
The following components were combined in a microfuge tube:
   "SiRNA Duplex molecule (diluted to 2 microMolar) 3.0 µL
   "Serum Free Media or PBS 51.0 µL

To the above mixture 54µl of 2x Lipofectamine solution was added 5 minutes prior to use.

Dendrimer plus siRNA Lipofectamine Complexes:
The following components were combined in a microfuge tube:
   four layer DNA dendrimer with or without Antibody (10ng/µL) 12.0 µL
   "SiRNA Duplex molecule (diluted to 2 microMolar) 3.0 µL
   "Serum Free Media or PBS 39.0 µL

This mixture was incubated at 37°C for 20-30 minutes and then place at room temperature until being combined with 2x Lipofectamine solution 5 minutes prior to use.

### Transfection Experiments

Transfection mixtures were introduced into wells in a tissue culture plate containing 2,000-10,000 live cells suitable as targets for *in-vitro* transfection and grown in the appropriate media containing 10% serum or in serum free media. Five microliters of the appropriate above formulation was added to 120uL of tissue culture media in a 96 well plate containing the plated cells. The final concentration of the siRNA was 2 nanomolar. Function of the siRNA was measured directly by quantifying the amount of intact target mRNA remaining in the cell after the addition of the Lipofectamine siRNA or Lipofectamine dendrimer-siRNA complex using a qRT-PCR assay designed to detect and the targeted mRNA (ssb) relative to an internal control mRNA (18s RNA and PPIB mRNA).

All knockdown determinations were relative to a Negative Control (no mRNA target) siRNA duplex containing the same structural modifications.

### List of experimental conditions tested:

Lipofectamine plus "SSB unmod" no dendrimer (#1), Lipofectamine plus "Neg unmod" no dendrimer (#2), Lipofectamine plus "SSB+26" no dendrimer (#3), Lipofectamine plus "Neg+26" no dendrimer (#4), Lipofectamine plus "Dendrimer no Ab plus "SSB unmod" (#5), Lipofectamine plus "Dendrimer no Ab plus "Neg unmod" (#6), Lipofectamine plus "Dendrimer no Ab plus "SSB+26" (#7), Lipofectamine plus "Dendrimer no Ab plus "Neg+26" (#8), Lipofectamine plus "720 biotin Dendrimer no Ab plus "SSB unmod" (#9), Lipofectamine plus "720 biotin Dendrimer no Ab plus "Neg unmod" (#10),Lipofectamine plus "720 biotin Dendrimer no Ab plus "SSB+26" (#11), Lipofectamine plus "720 biotin Dendrimer no Ab plus "Neg+26" (#12), Lipofectamine plus "720 biotin Dendrimer with Ab plus "SSB unmod" (#13), Lipofectamine plus "720 biotin Dendrimer with Ab plus "Neg unmod" (#14), Lipofectamine plus "720 biotin Dendrinier with Ab plus "SSB+26" (#15), Lipofectamine plus "720 biotin Dendrimer with Ab plus "Neg+26" (#16).

### Results

In all cases comparing the knockdown efficiency of "SSB unmod" to "SSB+26" regardless of whether the siRNA was combined with a dendrimer, little difference was observed between the two siRNA constructs, indicating that the 26 base extension was not impacting the results in either a positive or negative manor. Further, when the siRNA was attached to the dendrimer it performed as well as the unhybridized siRNA suggesting that the hybridized siRNA was efficiently released form the dendrimer siRNA construct.

Comparing knockdown efficiency of Lipofectamine siRNA complexes not containing, dendrimer (conditions #1-#4 above) to those (containing dendrimer (either #5-#8, #9-#12, or #13-#16) we observed a significant improvement in the efficiency of knockdown when dendrimer was present. Lipofectamine complexes with out dendrimer demonstrated about 80% knockdown compared to greater than 90-95% percent knockdown when dendrimers were present.

Little or no difference was observed comparing knockdown efficiency of Lipofectamine siRNA dendrimer complexes with or without antibody. Both were equally efficient.

While dendrimers containing biotin demonstrated a trend of better knockdown efficiency when combined with siRNA in Lipofectamine complexes compared to similar complexes prepared with dendrimers without biotin, no statistical difference of knockdown efficiency was observed at this dose of siRNA.

*Conclusion:* Based on the observed results it was concluded that DNA dendrimers when combined with liposomal transfection agents improve the mRNA knockdown efficiency of siRNA molecules. Based on the compositions used we theorize that dendrimers may operate to improve the release of the siRNA from subcellular compartments (e.g. endosomes).

## Claims

1. A composition comprising a DNA dendrimer attached to a siRNA molecule.

2. The composition of claim 1, further comprising a protein, a molecule containing a polyethylene glycol (PEG) moiety, biotin or a biotin derivative, fluorescein or a fluorescein derivative, or any combination thereof attached to said DNA dendrimer.

3. The composition of claim 2, comprising a) a DNA dendrimer attached to a siRNA molecule and an antibody or an antibody fragment, b) a DNA dendrimer attached to a siRNA molecule and biotin, or c) a DNA dendrimer attached to a siRNA molecule and fluorescein or a fluorescein derivative, including fluorescein isothiocyanate (FITC).

4. A method of preparing a composition comprising a DNA dendrimer and a siRNA molecule for de-novo knockdown of a transcribed target gene wherein the siRNA molecule comprises a sense strand and an antisense strand and wherein the method comprises the step of attaching the sense strand of said siRNA to said DNA dendrimer (a) via a disulfide bridging bond; (b) via the use of NHS ester dependent condensation reaction; (c) via the use of a bifunctional cross linking reaction, in particular a heterobifunctional cross linking reaction; or (d) via direct or indirect hybridization of the siRNA to DNA dendrimer sequence.

5. The method of claim 4, further comprising the step of attaching to said DNA dendrimer a protein, a peptide, an aptamer, fluorescein or a fluorescein derivative, a fluorescent dye, digoxigenin, cholesterol, a primary amine, a hydrocarbon spacer of length of 3 to 120 carbons, a molecule containing a polyethylene glycol (PEG) moiety, biotin or a biotin derivative, an antibody or antibody fragment, or any combination thereof.

6. A pharmaceutical composition comprising a composition according to any of claims 1 to 3 mixed with a pharmaceutically acceptable carrier.

7. A composition according to any of claims 1 to 3 or 6 for use as a medicament.

8. The composition according to claim 7, wherein the composition is to be transfected into cells.

9. Use of a composition according to claim 1 for in vitro transfection.

10. Use of a composition according to any of claims 1 to 3 for the manufacture of a medicament.

## Patentansprüche

1. Zusammensetzung, umfassend ein an ein siRNA-Molekül gebundenes DNA-Dendrimer.

2. Zusammensetzung nach Anspruch 1, ferner umfassend jeweils an das DNA-Dendrimer gebunden ein Protein, ein Molekül mit einer Polyethylenglykol(PEG)-Gruppierung, Biotin oder ein Biotinderivat, Fluorescein oder ein Fluoresceinderivat oder eine beliebige Kombination davon.

3. Zusammensetzung nach Anspruch 2, umfassend a) ein an ein siRNA-Molekül und einen Antikörper oder ein Antikörperfragment gebundenes DNA-Dendrimer, b) ein an ein siRNA-Molekül und Biotin gebundenes DNA-Dendrimer oder c) ein an ein siRNA-Molekül und Fluorescein oder ein Fluoresceinderivat, einschließlich Fluoresceinisothiocyanat (FITC), gebundenes DNA-Dendrimer.

4. Verfahren zur Herstellung einer ein DNA-Dendrimer und ein siRNA-Molekül umfassenden Zusammensetzung für das De-novo-Knockdown eines transkribierten Zielgens, wobei das siRNA-Molekül einen SenseStrang und einen Antisense-Strang umfasst und wobei in einem Schritt des Verfahrens der Sense-Strang des siRNA-Moleküls an das DNA-Dendrimer (a) über eine Disulfidbrückenbindung; (b) über die Verwendung einer NHS-Ester-abhängigen Kondensationsreaktion; (c) über die Verwendung einer bifunktionalen Vernetzungsreaktion, insbesondere einer heterobifunktionalen Vernetzungsreaktion; oder (d) über direkte oder indirekte Hybridisierung der siRNA an DNA-Dendrimersequenz gebunden wird.

5. Verfahren nach Anspruch 4, bei dem in einem weiteren Schritt an das DNA-Dendrimer ein Protein, ein Peptid, ein Aptamer, Fluorescein oder ein Fluoresceinderivat, ein Fluoreszenzfarbstoff, Digoxigenin, Cholesterin, ein primäres Amin, ein Kohlenwasserstoff-Spacer mit einer Länge von 3 bis 120 Kohlenstoffatomen, ein Molekül mit einer Polyethylenglykol(PEG)-Gruppierung, Biotin oder ein Biotinderivat, ein Antikörper oder Antikörperfragment oder eine beliebige Kombination davon gebunden wird.

6. Pharmazeutische Zusammensetzung, umfassend eine Zusammensetzung gemäß einem der Ansprüche 1 bis 3 im Gemisch mit einem pharmazeutisch unbedenklichen Trägerstoff.

7. Zusammensetzung gemäß einem der Ansprüche 1 bis 3 oder 6 zur Verwendung als Arzneimittel.

8. Zusammensetzung gemäß Anspruch 7, wobei die Zusammensetzung in Zellen zu transfizieren ist.

9. Verwendung einer Zusammensetzung gemäß Anspruch 1 zur In-vitro-Transfektion.

10. Verwendung einer Zusammensetzung gemäß einem der Ansprüche 1 bis 3 zur Herstellung eines Arzneimittels.

## Revendications

1. Composition comprenant un dendrimère d'ADN lié à une molécule d'ARNsi.

2. Composition de la revendication 1, comprenant en outre une protéine, une molécule contenant un fragment de polyéthylène glycol (PEG), de la biotine ou un dérivé de biotine, de la fluorescéine ou un dérivé de fluorescéine, ou une combinaison quelconque de ceux-ci liés audit dendrimère d'ADN.

3. Composition de la revendication 2, comprenant a) un dendrimère d'ADN lié à une molécule d'ARNsi est un anticorps ou un fragment d'anticorps, b) un dendrimère d'ADN lié à une molécule d'ARNsi et de la biotine, ou c) un dendrimère d'ADN lié à une molécule d'ARNsi et de la fluorescéine ou un dérivé de fluorescéine, comprenant l'isothiocyanate de fluorescéine (FITC).

4. Procédé de préparation d'une composition comprenant un dendrimère d'ADN et une molécule d'ARNsi pour l'inactivation de *novo* d'un gène cible transcrit, dans lequel la molécule d'ARNsi comprend un brin sens et un brin antisens et le procédé comprenant l'étape de liaison du brin sens dudit ARNsi audit dendrimère d'ADN (a) via une liaison de pont disulfure ; (b) via l'utilisation d'une réaction de condensation dépendante d'un ester de NHS ; (c) via l'utilisation d'une réaction de réticulation bifonctionnelle, en particulier une réaction de réticulation hétérobifonctionnelle ; ou (d) via hybridation directe ou indirecte de l'ARNsi à une séquence de dendrimère d'ADN.

5. Procédé de la revendication 4, comprenant en outre l'étape de liaison dudit dendrimère d'ADN à une protéine, un peptide, un aptamère, la fluorescéine ou un dérivé de fluorescéine, un colorant fluorescent, la digoxigénine, le cholestérol, une amine primaire, un espaceur hydrocarboné ayant une longueur de 3 à 120 carbones, une molécule contenant un fragment de polyéthylène glycol (PEG), la biotine ou un dérivé de biotine, un anticorps ou un fragment d'anticorps, ou une combinaison quelconque de ceux-ci.

6. Composition pharmaceutique comprenant une composition selon l'une quelconque des revendications 1 à 3 mélangée avec un véhicule pharmaceutiquement acceptable.

7. Composition selon l'une quelconque des revendications 1 à 3 ou 6 pour utilisation en tant que médicament.

8. Composition selon la revendication 7, la composition étant destinée à être transfectée dans des cellules.

9. Utilisation d'une composition selon la revendication 1 pour transfection *in vitro.*

10. Utilisation d'une composition selon l'une quelconque des revendications 1 à 3 pour la fabrication d'un médicament.
